# EUROPÄISCHE PATENTSCHRIFT

(11) **EP 2 787 736 B1**
(45) Veröffentlichungstag und Bekanntmachung des Hinweises auf die Patenterteilung: **25.05.2016**
(21) Anmeldenummer: 14162215.9
(22) Anmeldetag: 28.03.2014
(51) Int. Cl.: H04N 13/02, G02B 23/24, A61B 1/00, G01S 17/89

(54) **Kamera zur Erfassung von optischen Eigenschaften und von Raumstruktureigenschaften**
Camera for detecting optical characteristics and spatial structure characteristics
Caméra pour la détection de propriétés optiques et de propriétés de structure spatiale

(30) Priorität: 03.04.2013 DE 102013103333
(43) Veröffentlichungstag der Anmeldung: 08.10.2014
(73) Patentinhaber: Karl Storz GmbH & Co. KG, 78532 Tuttlingen (DE)
(72) Erfinder: Leonhard, Dr. Martin, 78532 Tuttlingen (DE); Krattiger, Beat, 78532 Tuttlingen (DE)

(56) Entgegenhaltungen:
- EP-A2- 2 451 150
- DE-A1-102006 017 003
- US-A1- 2007 158 770

## Beschreibung

Die vorliegende Erfindung ist auf eine Kameraeinrichtung, ein Endoskop oder Exoskop mit einer Kameraeinrichtung, ein Verfahren zum Herstellen einer Kameraeinrichtung und ein Verfahren zum Erfassen von optischen Eigenschaften und von Raumstruktureigenschaften eines Objekts gerichtet.

Moderne Endoskope sind, vor allem wenn sie eine Videokamera umfassen oder mit einer Videokamera gekoppelt sind, in der Lage, qualitativ hochwertige zweidimensionale Bilder aus dem Körperinneren eines Patienten oder aus einem Hohlraum in einer Maschine zu liefern. Für viele Anwendungen ist jedoch ein dreidimensionaler Seheindruck bzw. ein dreidimensionales Bild wünschenswert. Ein dreidimensionales Bild kann eine genauere Beurteilung von Abständen, Längen, Flächen, konvexen und konkaven Wölbungen und Volumina ermöglichen und damit auch eine sicherere Diagnose oder einen schnellen ökonomischen, sicheren und atraumatischen Eingriff unterstützen.

Ein stereoskopisches Endoskop, bei dem zwei von beabstandeten Punkten aus erfasste Bilder separat zum proximalen Ende übertragen werden, ist angesichts des erforderlichen Bauraums und der Tendenz zu immer kleineren Schaftquerschnitten allenfalls mit extremen Aufwand und bei Inkaufnahme anderer Nachteile realisierbar. Deshalb existiert eine Reihe von Ansätzen, ein hochwertiges zweidimensionales Bild und zusätzlich die Abstände der betrachteten Oberflächen vom distalen Ende des Endoskops zu erfassen. Unter Verwendung des zweidimensionalen Bilds und der Abstände kann ein Stereobild (mit je einem Teilbild für das linke und für das rechte Auge des Betrachters) berechnet werden, das dem Betrachter einen räumlichen Eindruck vermittelt. Besonders vorteilhaft ist, dass dazu unter Umständen ein herkömmliches oder nur leicht modifiziertes Endoskop verwendet werden kann und eine Modifikation nur an der Kameraeinrichtung erforderlich ist.

In DE 10 2006 017 003 A1 (Jochen Penne, FAU Erlangen) ist ein Endoskop zur Tiefendatenaquisition beschrieben. Eine Lichtquelle 102 sendet gemäß einer Modulationsinformation moduliertes Infrarot-Licht aus (Absätze [0046], [0051]). Ein Photonenmischdetektor 108 empfängt Licht und erzeugt ein das empfangene Licht repräsentierendes Sensorsignal. Das Sensorsignal wird unter Heranziehung der Modulationsinformation auf dem Prinzip des "time-of-flight" basierend ausgewertet, um Tiefendaten aus der Laufzeit des empfangenen Lichtsignals zu berechnen (Absätze [0018], [0030], [0042], [0047], [0048], [0049]). Neben einer Matrix von Tiefendaten bzw. Distanzwerten kann eine Matrix von Intensitätsinformationen bzw. -werten aus dem Sensorsignal erzeugt werden (Absatz [0018]). Eine weitere Lichtquelle 202 zur Erzeugung eines Tageslichtspektrums und ein weiterer Sensor 302 können zur Gewinnung von Bilddaten vorgesehen sein (Absätze [0023], [0054], [0062]). Alternativ erzeugt die Lichtquelle Licht im sichtbaren Spektralbereich und ein Beobachter nimmt die Szene in diesem Licht wahr (Absatz [0060]).

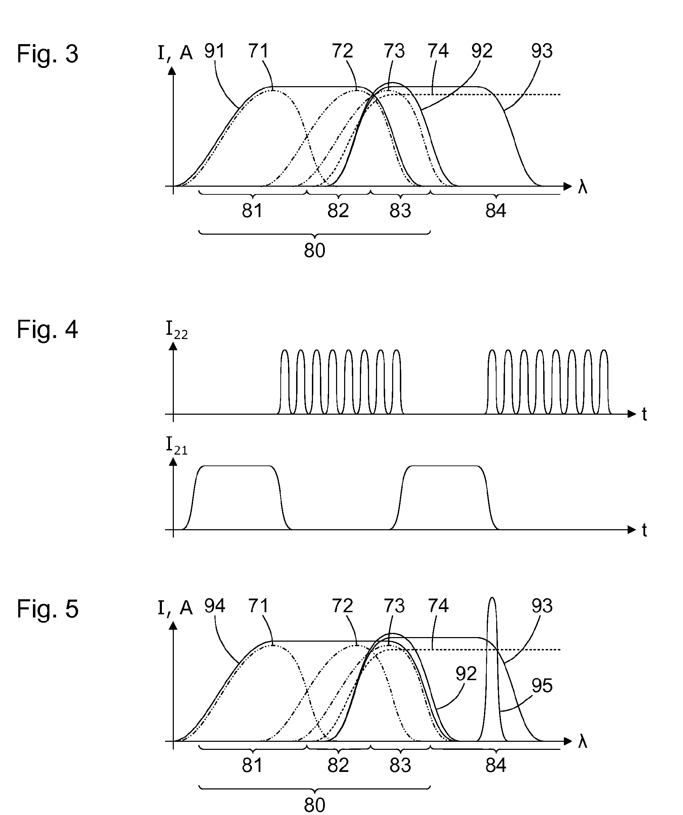

In DE 10 2007 006 351 A1 (Frederic Sarrat, Logitech) ist eine Bilderfassung beschrieben. Licht wird mittels eines Splitters geteilt und teils zu einem ersten Sensor zum Erfassen von Information in zwei Dimensionen und teils zu einem zweiten Sensor zum Erfassen von Information in einer dritten Dimension geleitet (Absätze [0011], [0012], [0036], [0040], [0041], [0046]). Ferner sind Sensoren beschrieben, die neben Pixeln für rotes (R), grünes (G) und blaues Licht (B) infrarotempfindliche Pixel (D) zur Abstandsmessung umfassen (Figuren 3A, 3B, 3C, Absätze [0034], [0035]).

In DE 10 2008 018 636 A1 (Beat Krattiger, Karl Storz) ist eine endoskopische 3D-Datenerfassung beschrieben. Mittels eines Strahlteilers wird von einem Objekt zurückkommende Strahlung auf einen von mehreren Bildsensoren abgebildet (Absatz [0018]). Ein Sensor ist phasenempfindlich. Wenn Bildsensoren verwendet werden, die unterschiedliche Sensorgrößen aufweisen, ist eine Anpassungsoptik zwischen dem Strahlteiler und mindestens einem Bildsensor angeordnet (Absatz [0023]). Aus einem 2D-Bild des beobachteten Bereichs und Entfernungsinformation wird ein Stereobild synthetisiert (Absätze [0036], [0037]).

In DE 10 2008 018 637 A1 (Beat Krattiger, Karl Storz) ist eine Fluoreszenz-Bildgebung mit einer (zeitlich) modulierten Fluoreszenzanregungsstrahlung beschrieben (Absätze [0011], [0012]). Fluoreszenzstrahlung wird auf einen phasenempfindlich ansteuerbaren Festkörpersensor abgebildet (Absätze [0019], [0020]). Mittels eines Strahlteilers wird (vom beleuchteten Gegenstand) empfangene Strahlung auf weitere Sensoren geleitet (Absätze [0031], [0032], [0033]). Im Fall von Sensoren unterschiedlicher Formate ist eine Anpassungsoptik vorgesehen (Absatz [0035]).

In der EP 2451150 A2 ist beschrieben, einen Time-of-Flight (TOF) Sensor zur Abstandserfassung und Bildsensoren in einer Kamera anzuordnen, oder TOF-Pixel und Farbpixel auf einem gemeinsamen Sensor anzuordnen. Die Abstandsinformation aus dem Signal des TOF-Sensors wird verwendet, um eine Farbkorrektur des durch den Bildsensor erfassten Bildes vorzunehmen. Des Weiteren ist beschrieben, einen TOF-Sensor in verschiedenen Bereichen mit Filtern zu versehen, um verschiedene Farbsignale und auch ein Abstandssignal mit einem einzelnen Sensor zu erfassen.

In der US 2007/158770 A1 ist ein spezieller Time-of-Flight Sensor beschrieben, der ein Intensitäts-und ein Abstandssignal liefern kann. Es wird vorgeschlagen dies zu nutzen, um ein dreidimensionales Farbbild zu erstellen.

Eine Aufgabe der vorliegenden Erfindung besteht darin, eine verbesserte Kameraeinrichtung, ein verbessertes Endoskop oder Exoskop, ein verbessertes Verfahren zum Herstellen einer Kameraeinrichtung und ein verbessertes Verfahren zum Erfassen von optischen Eigenschaften und von Raumstruktureigenschaften eines Objekts zu schaffen.

Diese Aufgabe wird durch die Gegenstände der unabhängigen Ansprüche gelöst.

Weiterbildungen sind in den abhängigen Ansprüchen angegeben.

Ausführungsbeispiele der vorliegenden Erfindung beruhen auf der Idee, mittels eines ToF-Bildsensors (ToF = Time of Flight), der phasenempfindlich ist und der nicht nur ein Abstands-Bild, sondern gleichzeitig ein Intensitäts-Bild in einem Farbkanal, das mit Intensitäts-Bildern in weiteren Farbkanälen zu einem Farbbild kombiniert wird, zu erfassen. Mit einem Intensitäts-Bild bzw. einem Intensitäts-Bildsignal wird ein Bild bzw. ein Bildsignal bezeichnet, das für ein (in der Regel rechteckiges) Raster von Bildpunkten bzw. Pixeln je einen Intensitäts- bzw. Helligkeitswert in einem Farbkanal (beispielsweise rot, grün oder blau) umfasst bzw. angibt. In der Regel werden je ein Intensitäts-Bild im roten, grünen bzw. blauen Wellenlängenbereich bzw. Farbkanal zu einem Farbbild zusammengesetzt. Ein Farbbild enthält beispielsweise für jedes Bildelement je einen Intensitäts- oder Helligkeitswert zu jedem Farbkanal. Mit einem Abstands-Bild bzw. einem Abstands-Bildsignal wird ein Bild bzw. ein Bildsignal bezeichnet, das für jedes Bildelement einen Abstand des auf das Bildelement bzw. den Pixel projizierten Oberflächenelements vom distalen Ende eines Endoskops oder vom Objektiv einer Kamera umfasst.

Die vorliegende Erfindung beruht auch auf der Erkenntnis, dass viele eigentlich im infraroten Wellenlängenbereich arbeitenden bzw. betriebenen ToF-Sensoren oder andere Abstands-Bildsensoren eine intrinsische Empfindlichkeit auch im roten Spektralbereich aufweisen. Diese Sensoren können deshalb gleichzeitig zur Erfassung eines Intensitäts-Bilds im roten Spektral- bzw. Wellenlängenbereich bzw. im roten Farbkanal genutzt werden. Zur Erfassung eines Farbbilds in drei Farbkanälen (insbesondere blau, grün und rot) und eines Abstands-Bilds sind also nicht vier Bildsensoren (je einer für blau, grün, rot und Abstand) erforderlich. Vielmehr reichen drei Bildsensoren aus, wenn mittels des Abstandssensors sowohl ein Abstands-Bildsignal als auch ein Intensitäts-Bildsignal zu einem der Farbkanäle erzeugt wird.

Eine Kameraeinrichtung zur Erfassung von optischen Eigenschaften in mehreren verschiedenen Wellenlängenbereichen und von Raumstruktureigenschaften eines Objekts umfasst einen Strahlteiler zur Auftrennung von Licht, das von einem Objekt kommt, in die mehreren verschiedenen Wellenlängenbereiche, einen ersten Bildsensor zum Erzeugen eines Sensorsignals, das auf einem vorbestimmten Wellenlängenbereich bezogen ist, wobei der erste Bildsensor aufgrund einer zeitlich modulierbaren Empfindlichkeit zur Erfassung eines Abstands eines Objekts von der Kameraeinrichtung aus einer Laufzeit von Licht ausgebildet ist, eine erste Signalverarbeitungseinrichtung zum Erzeugen eines ersten Intensitäts-Bildsignals und eines Abstands-Bildsignals unter Verwendung von Information über die zeitliche Modulation von Beleuchtungslicht und des ersten Sensorsignals, wobei das Abstands-Bildsignal räumliche Struktureigenschaften des Objekts darstellt, wenigstens einen zweiten Bildsensor zum Erzeugen eines zweiten Intensitäts-Bildsignals für einen zweiten vorbestimmten Wellenlängenbereich und eine zweite Signalverarbeitungseinrichtung zum Erzeugen eines Farb-Bildsignals aus dem ersten Intensitäts-Bildsignal und dem zweiten Intensitäts-Bildsignal, wobei das Farb-Bildsignal optische Eigenschaften des Objekts darstellt.

Die Kameraeinrichtung kann eine Kamera sein, die alle hier genannten Einrichtungen und Merkmale in einer einzigen Einheit, insbesondere innerhalb eines einzigen Gehäuses umfasst. Alternativ kann die Kameraeinrichtung mehrere Einheiten umfassen, die über elektrische, optische oder andere Signalleitungen miteinander verbunden sind. Insbesondere umfasst die Kameraeinrichtung eine Kamera mit dem Strahlteiler und den Bildsensoren, wobei die genannten und weitere Signalverarbeitungseinrichtungen jeweils in der Kamera oder in einer weiteren Einheit angeordnet sein können. Die Kameraeinrichtung ist insbesondere gemäß einem der unten beschriebenen Verfahren hergestellt und/oder zur Ausführung von einem der unten beschriebenen Verfahren zum Erfassen von optischen Eigenschaften und von Raumstruktureigenschaften eines Objekts ausgebildet. Die Kameraeinrichtung kann teilweise oder vollständig in ein Endoskop oder in ein Exoskop integriert oder zur Kombination mit einem Endoskop oder mit einem Exoskop ausgebildet sein.

Die Kameraeinrichtung ist insbesondere zur gleichzeitigen oder im Wesentlichen gleichzeitigen Erfassung von optischen Eigenschaften und von Raumstruktureigenschaften eines Objekts ausgebildet. Optische Eigenschaften und Raumstruktureigenschaften eines Objekts werden auch im Wesentlichen gleichzeitig erfasst, wenn (insbesondere mit einer der üblichen Bildwiederholfrequenzen im Bereich von 25 Bildern pro Sekunde bis 100 Bildern pro Sekunde) alternierend optische Eigenschaften und Raumstruktureigenschaften erfasst werden.

Optische Eigenschaften in mehreren verschiedenen Wellenlängenbereichen eines Objekts sind insbesondere der Remissionsgrad, der Reflexionsgrad oder Fluoreszenzeigenschaften in den mehreren verschiedenen Wellenlängenbereichen des Objekts.

Raumstruktureigenschaften des Objekts sind insbesondere Abstände von Oberflächenbereichen des Objekts, die auf die Bildelemente des ersten Bildsensors abgebildet werden, vom ersten Bildsensor, gemessen jeweils entlang des optischen Pfads zwischen Objekt und Bildsensor. Die Weglängen entlang des optischen Pfads innerhalb der Kamera und ggf. innerhalb eines Endoskops sind konstant. Deshalb unterscheiden sich Abstände des Objekts bzw. seiner Oberflächenbereiche vom ersten Bildsensor und Abstände des Objekts bzw. seiner Oberflächenbereiche von der Frontlinse der Kameraeinrichtung oder vom Lichteintrittsfenster eines Endoskops, das mit der Kameraeinrichtung optisch gekoppelt ist, lediglich durch einen vorbestimmten Summanden. Deshalb wird nachfolgend nicht mehr ausdrücklich erwähnt, auf welchen Bezugspunkt oder welche Bezugsebene die Abstände bezogen sind.

Die Kameraeinrichtung ist zur ortsaufgelösten Erfassung der optischen Eigenschaften und der Raumstruktureigenschaft ausgebildet. Insbesondere ist die Kameraeinrichtung zur Erzeugung eines mehrfarbigen Bilds des Objekts in den mehreren verschiedenen Wellenlängenbereichen bzw. Farbkanälen (beispielsweise rot, grün, blau) und zur Erzeugung eines Abstands-Bilds ausgebildet. Das mehrfarbige Bild enthält für jeden einer Mehrzahl von (insbesondere in einem rechteckigen Raster angeordneten) Bildelementen oder Pixeln je einen Intensitäts- oder Helligkeitswert für jeden Farbkanal bzw. für jeden Wellenlängenbereich. Das Abstands-Bild umfasst für jedes einer Mehrzahl von (insbesondere in einem rechteckigen Raster angeordneten) Bildelementen bzw. Pixeln oder Pixelgruppen einen Abstandswert. Die Auflösung des Abstands-Bilds kann hierbei geringer sein als die Auflösung des mehrfarbigen Bilds.

Der Strahlteiler kann jedes optische Element sein, das geeignet ist einfallendes Licht in verschiedene Wellenlängen bzw. Wellenlängenbereiche aufzuteilen. Er umfasst insbesondere ein oder mehrere Prismen. An deren Oberflächen oder innerhalb derer können ebene oder gekrümmte dichroitisch (d. h. wellenlängenabhängig) reflektierende Schichten vorgesehen sein. Der erste Bildsensor, der zweite Bildsensor und optional ein dritter Bildsensor und gegebenenfalls weitere Bildsensoren sind jeweils einem der mehreren verschiedenen Wellenlängenbereiche zugeordnet. Alternativ kann ein Bildsensor, insbesondere der zweite Bildsensor, mehreren Wellenlängenbereichen zugeordnet sein. Beispielsweise umfasst der zweite Bildsensor eine alternierende Anordnung von Bildelementen bzw. Pixeln, die zwei verschiedenen Wellenlängenbereichen zugeordnet sind und dazu mit Farbfiltern zweier verschiedener Arten versehen sind.

Die Zuordnung eines Bildsensors zu einem Wellenlängenbereich (oder zu mehreren Wellenlängenbereichen) kann sich aus der Anordnung des Bildsensors an einem bestimmten Ort relativ zum Strahlteiler, insbesondere an einer bestimmten Lichtaustrittsfläche eines Strahlteiler-Prismas ergeben, an den bzw. die lediglich Licht in dem zugeordneten Wellenlängenbereich gelangt. Alternativ oder zusätzlich kann ein Bildsensor jeweils ausschließlich oder überwiegend für Licht in dem zugeordneten Wellenlängenbereich empfindlich sein. Jeder Bildsensor kann ein ortsempfindlicher bzw. ortsauflösender Halbleitersensor (beispielsweise CMOS oder CCD) sein.

Der erste Bildsensor ist ein phasenempfindlicher Detektor, z.B. ein ToF-Sensor. Der erste Bildsensor kann zum Erzeugen eines ersten Sensorsignals, das ausschließlich auf den ersten vorbestimmten Wellenlängenbereich (insbesondere auf den roten Bereich innerhalb des für das menschliche Auge sichtbaren Wellenlängenbereichs) bezogen ist, ausgebildet sein. Alternativ kann das erste Sensorsignal ferner auf einen weiteren Wellenlängenbereich (beispielsweise im nahen oder mittleren Infrarot) bezogen sein.

Der erste Bildsensor ist insbesondere aufgrund einer zeitlich hochfrequent (mehrere 10 MHz, mehrere 100 MHz oder mehr) modulierbaren Empfindlichkeit zur Erfassung von Abständen geeignet. Bei Beleuchtung des Objekts mit Beleuchtungslicht, das mit einer insbesondere hohen Frequenz moduliert ist und Modulation der Empfindlichkeit des ersten Bildsensors mit derselben Frequenz entsteht an jedem Bildelement des ersten Bildsensors ein Signal, dessen Phase vom Abstand des Objekts (entlang des optischen Pfads) vom ersten Bildsensor abhängt.

Der zweite Bildsensor ist insbesondere zur Erzeugung eines Sensorsignals, das ein zweites Intensitäts-Bildsignal für den blauen Wellenlängenbereich umfasst, vorgesehen und ausgebildet. Ferner ist insbesondere ein dritter Bildsensor zum Erzeugen eines Sensorsignals, das ein drittes Intensitäts-Bildsignal für den grünen Wellenlängenbereich umfasst, vorgesehen und ausgebildet. Ferner können weitere Bildsensoren für weitere Farbkanäle im ultravioletten, infraroten oder sichtbaren Wellenlängenbereich vorgesehen sein.

Alternativ kann der zweite Bildsensor zur Erzeugung eines Sensorsignals neben dem zweiten Intensitäts-Bildsignal für den blauen Wellenlängenbereich ein drittes Intensitäts-Bildsignal für den grünen Wellenlängenbereich erfassen. Insbesondere ist der zweite Bildsensor ein Bayer-Sensor oder ein anderer Bildsensor mit zwei (oder mehr) Gruppen von Bildelementen bzw. Pixeln, vor denen Farbfilter in zwei (oder mehr) verschiedenen Farben angeordnet sind.

Die erste Signalverarbeitungseinrichtung, die zweite Signalverarbeitungseinrichtung sowie weitere, im Folgenden genannte oder nicht genannte Signalverarbeitungseinrichtungen können jeweils als diskrete analoge oder insbesondere digitale Schaltungen, als Teile von integrierten Schaltungen als FPGA (field programmable gate array) mit einer vorbestimmten Konfiguration oder als Teil einer FPGA, als Prozessor oder Computer mit Firmware und/oder Software, als Firmware oder Software oder Teil einer Firmware oder Software für einen Prozessor oder Computer oder als eine Kombination derselben ausgebildet sein.

Die erste Signalverarbeitungseinrichtung ist insbesondere zum Erzeugen eines ersten IntensitätsBildsignals, das keine Abstandsinformation enthält, ausgebildet. Dabei ist eine mit zunehmendem Abstand eines Objekts von der Kameraeinrichtung aufgrund abnehmender Beleuchtungsstärke abnehmende Helligkeit keine Abstandsinformation im Sinne der vorliegenden Beschreibung.

Die erste Signalverarbeitungseinrichtung ist insbesondere ferner ausgebildet, um das Abstands-Bildsignal unter Verwendung von Information über die Phase des Bildsignals bezüglich einer periodischen Modulation von Beleuchtungslicht zu erzeugen. Die erste Signalverarbeitungseinrichtung kann teilweise oder vollständig in den ersten Bildsensor integriert sein.

Die zweite Signalverarbeitungseinrichtung ist insbesondere ausgebildet, um das Farb-Bildsignal aus dem mittels des ersten Bildsensors und der ersten Signalverarbeitungseinrichtung erzeugten ersten Intensitäts-Bildsignal, dem zweiten Intensitäts-Bildsignal vom zweiten Bildsensor und einem dritten Intensitäts-Bildsignal von einem dritten Bildsensor zu erzeugen. Alternativ können sowohl das zweite Intensitäts-Bildsignal als auch das dritte Intensitäts-Bildsignal (und optional weitere Intensitäts-Bildsignale) vom zweiten Bildsensor herrühren. In diesem Fall ist der zweite Bildsensor insbesondere ein Bayer-Sensor mit einer schachbrettartigen Anordnung von Farbfiltern vor einer entsprechenden Anordnung lichtempfindlicher Zellen des Bildsensors.

Das vom ersten Bildsensor herrührende Intensitäts-Bildsignal kann das einzige von der zweiten Signalverarbeitungseinrichtung zur Erzeugung des Farb-Bildsignals verwendete Intensitäts-Bildsignal sein, das auf einen bestimmten Wellenlängenbereich bezogen ist (insbesondere auf den roten Wellenlängenbereich). Alternativ kann die zweite Signalverarbeitungseinrichtung ausgebildet sein, um das erste Intensitäts-Bildsignal vom ersten Bildsensor mit einem weiteren Intensitäts-Bildsignal im gleichen oder in einem überlappenden Wellenlängenbereich zu kombinieren. Eine Kombination der auf gleiche oder überlappende Wellenlängenbereiche bezogenen Intensitäts-Bildsignale vom ersten Bildsensor und von einem weiteren Bildsensor kann eine Reduzierung von Farbrauschen, eine Verbesserung der Farbwiedergabe oder eine verbesserte Differenzierung zwischen malignem und benignem Gewebe ermöglichen.

Die doppelte Nutzung des ersten Bildsensors sowohl zur Erzeugung eines Abstands-Bildsignals als auch zur Erzeugung eines Intensitäts-Bildsignals im ersten vorbestimmten Wellenlängenbereich kann somit eine Reduzierung der Anzahl der insgesamt verwendeten Bildsensoren und/oder eine Verbesserung der Farbwiedergabe ermöglichen und/oder eine Unterscheidung von benignem und malignem Gewebe unterstützen.

Bei einer Kameraeinrichtung, wie sie hier beschrieben ist, liegt der erste Wellenlängenbereich insbesondere zumindest teilweise innerhalb des für das menschliche Auge sichtbaren Wellenlängenbereichs.

Für das menschliche Auge ist elektromagnetische Strahlung mit Wellenlängen im Bereich von ca. 380 nm bis ca. 750 nm sichtbar. Der erste Wellenlängenbereich umfasst insbesondere vom menschlichen Auge als rot wahrgenommene Wellenlängen (ca. 600 nm bis 750 nm).

Bei einer Kameraeinrichtung, wie sie hier beschrieben ist, liegt der erste Wellenlängenbereich insbesondere zumindest teilweise innerhalb des infraroten Wellenlängenbereichs (Wellenlängen größer als 750 nm).

Bei einer Kameraeinrichtung, wie sie hier beschrieben ist, ist die zweite Signalverarbeitungseinrichtung insbesondere ausgebildet, um das Farb-Bildsignal ohne Verwendung des Abstands-Bildsignals zu erzeugen.

Die zweite Signalverarbeitungseinrichtung erzeugt also nicht etwa ein Bildsignal für ein Bild, bei dem beispielsweise Abstände durch Farben codiert sind. Vielmehr ist die zweite Signalverarbeitungseinrichtung ausgebildet, um ein Farb-Bildsignal zu erzeugen, das - von der mit zunehmenden Abstand des Objekts abnehmenden Bildhelligkeit abgesehen - keine Information über den Abstand enthält und ohne Verwendung von Information über Abstände erzeugt ist.

Eine Kameraeinrichtung, wie sie hier beschrieben ist, ist insbesondere durch Entfernen eines Bildsensors am Strahlteiler und Ersetzen des entfernten Bildsensors durch den ersten Bildsensor hergestellt.

Die Herstellung der Kameraeinrichtung umfasst neben den Schritten des Entfernens und des Ersetzens weitere, hier nicht genannte Schritte.

Eine Kameraeinrichtung, wie sie hier beschrieben ist, umfasst insbesondere ferner eine dritte Signalverarbeitungseinrichtung zum Erzeugen eines Stereo-Bildsignals.

Die dritte Signalverarbeitungseinrichtung ist insbesondere ausgebildet, um das Farb-Bildsignal und das Abstands-Bildsignal zu empfangen und zu verwenden, um das Stereo-Bildsignal zu erzeugen. Das Stereo-Bildsignal ist zur Steuerung einer Wiedergabe von zwei unterschiedlichen Bildern, die für je ein Auge eines Betrachters vorgesehen sind, ausgebildet. Die dritte Signalverarbeitungseinrichtung erzeugt das Stereo-Bildsignal insbesondere durch eine Verschiebung von Bildelementen des Farb-Bildsignals abhängig von den zugeordneten Abständen in für beide Augen entgegengesetzte Richtungen.

Bei einer Kameraeinrichtung, wie sie hier beschrieben ist, weisen der erste Bildsensor und der zweite Bildsensor insbesondere unterschiedliche Auflösungen auf, wobei die Kameraeinrichtung ferner eine Signalverarbeitungseinrichtung zum Anpassen einer ersten Auflösung des ersten Intensitäts-Bildsignals und einer zweiten Auflösung des zweiten Intensitäts-Bildsignals aneinander umfasst.

Diese weitere Signalverarbeitungseinrichtung kann mit dem ersten Bildsensor und/oder mit der ersten Signalverarbeitungseinrichtung und/oder mit der zweiten Signalverarbeitungseinrichtung teilweise oder vollständig integriert oder identisch sein. Die weitere Signalverarbeitungseinrichtung ist insbesondere vorgesehen und ausgebildet, um die Auflösung desjenigen Intensitäts-Bildsignals mit einer geringeren Auflösung an die Auflösung desjenigen Intensitäts-Bildsignals mit einer höheren Auflösung anzupassen.

In vielen Fällen weisen Bildsensoren, die aufgrund ihrer zeitlich hochfrequent modulierbaren Empfindlichkeit zur Erfassung von Abständen geeignet sind, eine vergleichsweise geringe oder sehr geringe Auflösung auf. Deshalb hat beispielsweise der erste Bildsensor nur jeweils wenige 100 Spalten und Zeilen von Bildelementen. Der zweite Bildsensor und ggf. der dritte Bildsensor weisen hingegen beispielsweise jeweils eine Auflösung von 1920 x 1080 Bildelementen auf.

Bei einer Kameraeinrichtung, wie sie hier beschrieben ist, ist die weitere Signalverarbeitungseinrichtung insbesondere ausgebildet, um zum Anpassen verschiedener Auflösungen das Intensitäts-Bildsignal mit der geringeren Auflösung für Positionen von Bildelementen des Intensitäts-Bildsignals mit der höheren Auflösung zu interpolieren.

Insbesondere ist die weitere Signalverarbeitungseinrichtung ausgebildet, um zum Interpolieren Information aus dem Intensitäts-Bildsignal mit der höheren Auflösung zu verwenden.

Insbesondere wird das vom ersten Bildsensor herrührende erste Intensitäts-Bildsignal (das insbesondere ausschließlich, überwiegend oder zumindest teilweise auf den roten Wellenlängenbereich bezogen ist) für Positionen von Bildelementen des vom zweiten Bildsensor herrührenden zweiten Intensitäts-Bildsignals (insbesondere bezogen auf den grünen Wellenlängenbereich) und gegebenenfalls des vom dritten Bildsensor herrührenden dritten Intensitäts-Bildsignals (insbesondere bezogen auf den blauen Wellenlängenbereich) interpoliert. Bei der Interpolation wird insbesondere Information über Kanten und andere Strukturen, die im zweiten Intensitäts-Bildsignal und ggf. im dritten Intensitäts-Bildsignal enthalten ist, verwendet. Beispielsweise werden Intensitäts- bzw. Helligkeitswerte des zweiten Intensitäts-Bildsignals und ggf. des dritten und weiterer Intensitäts-Bildsignale durch Splines bzw. Polynome angenähert, deren Koeffizienten dann zur Interpolation des ersten Intensitäts-Bildsignals verwendet werden.

Bei einer Kameraeinrichtung, wie sie hier beschrieben ist, weisen der erste Bildsensor und der zweite Bildsensor insbesondere unterschiedliche Abmessungen auf, wobei die Kameraeinrichtung ferner eine Einrichtung zur Anpassung der Sensorgrößen aneinander umfasst.

Die Einrichtung zur Anpassung der Sensorgrößen umfasst insbesondere eine Anordnung von einer oder mehreren Linsen zwischen dem Strahlteiler und dem ersten Bildsensor und/oder eine Anordnung von einer oder mehreren Linsen zwischen dem Strahlteiler und dem zweiten Bildsensor, um die Raumwinkelbereiche, innerhalb derer Objekte auf den ersten Bildsensor bzw. auf den zweiten Bildsensor abgebildet werden, aneinander anzupassen. Im Fall von beim ersten Bildsensor und beim zweiten Bildsensor unterschiedlicher Verhältnisse zwischen Breite und Höhe ist die Einrichtung zur Anpassung der Sensorgrößen für eine anamorphe Abbildung ausgebildet bzw. ist oder umfasst einen Anamorphoten.

Ein Endoskop oder Exoskop umfasst eine Kameraeinrichtung, wie sie hier beschrieben ist.

Die Kameraeinrichtung kann dauerhaft bzw. nicht ohne Verwendung von Werkzeugen zerstörungsfrei trennbar mit dem Endoskop oder Exoskop verbunden sein. Insbesondere kann die Kameraeinrichtung am proximalen oder am distalen Ende eines Endoskops vorgesehen sein. Alternativ kann das Endoskop oder Exoskop so ausgebildet sein, dass die Kameraeinrichtung ohne Verwendung von Werkzeug zerstörungsfrei abtrennbar ist. Ein Exoskop ist im Gegensatz zu einem Endoskop nicht dafür vorgesehen, in einen Hohlraum im Körper eines Patienten oder in einer Maschine eingeführt zu werden. Stattdessen ist ein Exoskop typischerweise dafür vorgesehen und ausgebildet, in einem Abstand von einigen oder wenigen Dezimetern oder von einigen oder wenigen Zentimetern außerhalb des Körpers bzw. der Maschine angeordnet zu werden. Ein Exoskop bildet insbesondere eine extrakorporale Visualisierungseinrichtung für chirurgische Eingriffe.

Ein Verfahren zum Herstellen einer Kameraeinrichtung zur Erfassung von optischen Eigenschaften in mehreren verschiedenen Wellenlängenbereichen und von Raumstruktureigenschaften eines Objekts umfasst ein Bereitstellen eines Strahlteilers zur Auftrennung von Licht in mehrere verschiedene Wellenlängenbereiche, wobei an dem Strahlteiler für jeden der mehreren verschiedenen Wellenlängenbereiche je ein lichtempfindlicher Bildsensor vorgesehen ist; ein Entfernen eines Bildsensors von dem Strahlteiler; und ein Ersetzen des entfernten Bildsensors durch einen ersten Bildsensor, der aufgrund zeitlich modulierbarer Empfindlichkeit zur Erfassung eines Abstands eines Objekts von der Kameraeinrichtung aus einer Laufzeit von Licht ausgebildet ist; wobei ein zweiter Bildsensor am Strahlteiler verbleibt.

Das Verfahren ist insbesondere zum Herstellen einer Kameraeinrichtung, wie sie hier beschrieben ist, ausgebildet. Insoweit wird auf die obige Darstellung von Merkmalen, Eigenschaften, Funktionen und Vorteilen einer Kameraeinrichtung verwiesen. Das Verfahren ist insbesondere zum Herstellen einer Kameraeinrichtung zur Durchführung eines Verfahrens zur Erfassung von optischen Eigenschaften in mehreren verschiedenen Wellenlängenbereichen und von Raumstruktureigenschaften eines Objekts, wie es unten beschrieben ist, ausgebildet. Insoweit wird auf die Darstellung von Merkmalen, Eigenschaften, Wirkungen und Vorteilen des Verfahrens weiter unten verwiesen.

Am Strahlteiler ist insbesondere an drei Lichtaustrittsflächen je ein Bildsensor vorgesehen, je einer für den blauen, für den grünen und für den roten Wellenlängenbereich. Alternativ kann der Strahlteiler lediglich zwei Lichtaustrittsflächen und zwei Bildsensoren aufweisen. Beispielsweise ist ein Bildsensor dem roten Wellenlängenbereich und ein Bildsensor dem grünen und dem blauen Wellenlängenbereich zugeordnet. Der dem grünen und dem blauen Wellenlängenbereich zugeordnete Bildsensor kann ausgebildet sein, um gleichzeitig oder abwechselnd Intensitäts-Bildsignale (die ineinander verschränkt sein können) zum grünen und zum blauen Wellenlängenbereich zu erzeugen. Alternativ kann der Strahlteiler vier oder mehr Lichtaustrittsflächen mit je einem Bildsensor für je einen Wellenlängenbereich aus einer entsprechenden Anzahl verschiedener Wellenlängenbereiche aufweisen.

Der entfernte Bildsensor ist insbesondere dem vom menschlichen Auge als rot wahrgenommenen Wellenlängenbereich (ca. 600 nm bis ca. 750 nm) zugeordnet.

Insbesondere Strahlteiler mit drei Lichtaustrittsflächen und drei Bildsensoren sind in großer Vielfalt und teils zu günstigen Preisen verfügbar, beispielsweise als hochwertige Videokameras oder für hochwertige Videokameras. Einen der Bildsensoren vom Strahlteiler zu entfernen und durch einen zur Erfassung von Abständen geeigneten Bildsensor zu ersetzen, kann einen besonders kostengünstigen Weg zur Herstellung einer Kameraeinrichtung mit den gewünschten Eigenschaften darstellen.

Bei einem Verfahren zum Herstellen, wie es hier beschrieben ist, ist der erste Bildsensor insbesondere in dem Wellenlängenbereich, der dem entfernten Bildsensor zugeordnet war, empfindlich.

Der dem entfernten Bildsensor zugeordnete Wellenlängenbereich ist insbesondere der vom menschlichen Auge als rot wahrgenommene Wellenlängenbereich. Alternativ kann der dem entfernten Bildsensor zugeordnete Wellenlängenbereich beispielsweise ein vom menschlichen Auge als blau und grün wahrgenommener Wellenlängenbereich sein. Sowohl der entfernte Bildsensor als auch der diesen ersetzende erste Bildsensor kann jeweils zusätzlich in weiteren Wellenlängenbereichen empfindlich sein, beispielsweise im infraroten.

Bei einem Verfahren zum Herstellen, wie es hier beschrieben ist, wird insbesondere ferner eine erste Signalverarbeitungseinrichtung zum Empfangen eines ersten Sensorsignals von dem ersten Bildsensor und zum Erzeugen eines ersten Intensitäts-Bildsignals und eines Abstands-Bildsignals unter Verwendung des ersten Sensorsignals des ersten Bildsensors bereitgestellt.

Das Bereitstellen umfasst insbesondere ein Koppeln bzw. wirksames Verbinden der ersten Signalverarbeitungseinrichtung mit dem ersten Bildsensor mittels elektrischer, optischer oder anderer Signalleitungen zum Übertragen analoger oder digitaler Signale vom ersten Bildsensor zur ersten Signalverarbeitungseinrichtung. Die erste Signalverarbeitungseinrichtung kann mit dem ersten Bildsensor teilweise oder vollständig integriert sein. In diesem Fall umfasst der Schritt des Ersetzens des entfernten Bildsensors durch den ersten Bildsensor das Bereitstellen der ersten Signalverarbeitungseinrichtung.

Die erste Signalverarbeitungseinrichtung ist insbesondere (wie oben bereits für die Kameraeinrichtung beschrieben) zum Erzeugen des Abstands-Bildsignals unter Verwendung von Information über eine zeitliche Modulation des Beleuchtungslichts ausgebildet.

Bei einem Verfahren zum Herstellen, wie es hier beschrieben ist, wird ferner insbesondere eine zweite Signalverarbeitungseinrichtung zum Empfangen des ersten Intensitäts-Bildsignals, zum Empfangen eines zweiten Intensitäts-Bildsignals für einen zweiten vorbestimmten Wellenlängenbereich von dem zweiten Bildsensor und zum Erzeugen eines Farb-Bildsignals unter Verwendung des ersten Intensitäts-Bildsignals und des zweiten Intensitäts-Bildsignals bereitgestellt.

Die zweite Signalverarbeitungseinrichtung ist insbesondere ferner zum Empfangen eines dritten Sensorsignals, das ein drittes Intensitäts-Bildsignal für einen dritten vorbestimmten Wellenlängenbereich umfasst, von einem dritten Bildsensor am Strahlteiler und zum Erzeugen eines Farb-Bildsignals auch unter Verwendung des dritten Intensitäts-Bildsignals ausgebildet. Alternativ ist die zweite Signalverarbeitungseinrichtung ausgebildet, um vom zweiten Bildsensor ein Sensorsignal zu empfangen, das das zweite Intensitäts-Bildsignal und ein drittes Intensitäts-Bildsignal für einen dritten vorbestimmten Wellenlängenbereich und optional ein viertes oder weitere Intensitäts-Bildsignale für einen vierten oder weitere vorbestimmten Wellenlängenbereiche umfasst. Ferner kann die zweite Signalverarbeitungseinrichtung zum Empfangen weiterer Sensorsignale, die weitere Intensitäts-Bildsignale umfassen, und zum Erzeugen des Farbbilds auch unter Verwendung dieser weiteren Intensitäts-Bildsignale ausgebildet sein.

Das Bereitstellen der zweiten Signalverarbeitungseinrichtung umfasst insbesondere ein Koppeln bzw. ein wirksames Verbinden der zweiten Signalverarbeitungseinrichtung mit der ersten Signalverarbeitungseinrichtung, mit dem zweiten Bildsensor und ggf. mit weiteren Bildsensoren mittels elektrischer, optischer oder anderer Signalleitungen zum Übertragen analoger oder digitaler Signale.

Bei einem Verfahren zum Herstellen, wie es hier beschrieben ist, ist die zweite Signalverarbeitungseinrichtung insbesondere ausgebildet, um das Farb-Bildsignal ohne Verwendung des Abstands-Bildsignals zu erzeugen.

Bei einem Verfahren zum Herstellen, wie es hier beschrieben ist, wird die zweite Signalverarbeitungseinrichtung insbesondere so mit der ersten Signalverarbeitungseinrichtung gekoppelt, dass die zweite Signalverarbeitungseinrichtung das Abstands-Bildsignal nicht empfängt.

Bei einem Verfahren zum Herstellen, wie es hier beschrieben ist, wird insbesondere ferner eine dritte Signalverarbeitungseinrichtung zum Empfangen des Farb-Bildsignals, zum Empfangen des Abstands-Bildsignals und zum Erzeugen eines Stereo-Bildsignals unter Verwendung des Farb-Bildsignals und des Abstands-Bildsignals bereitgestellt.

Das Bereitstellen der dritten Signalverarbeitungseinrichtung umfasst insbesondere ein Koppeln bzw. wirksames Verbinden der dritten Signalverarbeitungseinrichtung mit der ersten Signalverarbeitungseinrichtung und mit der zweiten Signalverarbeitungseinrichtung mittels elektrischer, optischer oder anderer Signalleitungen zum Übertragen analoger oder digitaler Signale.

Bei einem Verfahren zum Herstellen, wie es hier beschrieben ist, wird ferner insbesondere eine weitere Signalverarbeitungseinrichtung zum Anpassen einer ersten Auflösung des ersten Intensitäts-Bildsignals und einer zweiten Auflösung des zweiten Intensitäts-Bildsignals aneinander bereitgestellt.

Das Bereitstellen der weiteren Signalverarbeitungseinrichtung umfasst insbesondere ein Koppeln bzw. wirksames Verbinden der weiteren Signalverarbeitungseinrichtung mit der ersten Signalverarbeitungseinrichtung, mit der zweiten Signalverarbeitungseinrichtung und optional mit dem zweiten Bildsensor und ggf. und optional mit einem oder mehreren weiteren Bildsensoren mittels elektrischer, optischer oder anderer Signalleitungen zum Übertragen von analogen oder digitalen Signalen.

Bei einem Verfahren zum Herstellen, wie es hier beschrieben ist, wird insbesondere eine weitere Signalverarbeitungseinrichtung zum Anpassen von Auflösungen bereitgestellt, die ausgebildet ist, ein Intensitäts-Bildsignal mit einer geringeren Auflösung für Positionen von Bildelementen eines Intensitäts-Bildsignals mit einer höheren Auflösung zu interpolieren.

Die bereitgestellte weitere Signalverarbeitungseinrichtung ist insbesondere ausgebildet, um zum Interpolieren Information aus dem Intensitäts-Bildsignal mit der höheren Auflösung zu verwenden.

Jeder der Schritte des Bereitstellens der ersten Signalverarbeitungseinrichtung, des Bereitstellens der zweiten Signalverarbeitungseinrichtung, des Bereitstellens der dritten Signalverarbeitungseinrichtung und des Bereitstellens der weiteren Signalverarbeitungseinrichtung kann teilweise oder vollständig gleichzeitig mit einem Bereitstellen des ersten Bildsensors bzw. mit dem Schritt des Ersetzens des entfernten Bildsensors durch den ersten Bildsensor gleichzeitig ausgeführt werden. Insbesondere sind die erste Signalverarbeitungseinrichtung, die zweite Signalverarbeitungseinrichtung, die dritte Signalverarbeitungseinrichtung und die weitere Signalverarbeitungseinrichtung jeweils teilweise oder vollständig mit dem ersten Bildsensor in einem Bauteil integriert.

Ein Verfahren zum Erfassen von optischen Eigenschaften in mehreren verschiedenen Wellenlängenbereichen und von Raumstruktureigenschaften eines Objekts umfasst ein Beleuchten des Objekts mit in vorbestimmter Weise zeitlich moduliertem Beleuchtungslicht; ein Erfassen eines ersten Sensorsignals, das auf einen ersten vorbestimmten Wellenlängenbereich bezogen ist, mittels eines ersten Bildsensors mit zeitlich modulierter Empfindlichkeit; ein Erzeugen eines ersten Intensitäts-Bildsignals unter Verwendung des ersten Sensorsignals; ein Erzeugen eines Abstands-Bildsignals unter Verwendung von Information über die zeitliche Modulation des Beleuchtungslichts und des ersten Sensorsignals; ein Erfassen eines zweiten Intensitäts-Bildsignals, das auf einen zweiten vorbestimmten Wellenlängenbereich bezogen ist, mittels eines zweiten Bildsensors; und ein Erzeugen eines Farb-Bildsignals unter Verwendung des ersten Intensitäts-Bildsignals und des zweiten Intensitäts-Bildsignals; wobei das Farb-Bildsignal optische Eigenschaften des Objekts und das Abstands-Bildsignal Raumstruktureigenschaften des Objekts darstellen.

Das Verfahren ist insbesondere zum endoskopischen und insbesondere zum gleichzeitigen oder im Wesentlichen gleichzeitigen Erfassen von optischen Eigenschaften in mehreren verschiedenen Wellenlängenbereichen und von Raumstruktureigenschaften eines Objekts ausgebildet. Das Verfahren ist insbesondere mittels einer Kameraeinrichtung, wie sie hier beschrieben ist, oder mittels einer Kameraeinrichtung, die mit einem Verfahren zum Herstellen, wie es hier beschrieben ist, hergestellt ist, ausführbar. Insoweit wird auf die Darstellung von Merkmalen, Eigenschaften, Funktionen, Wirkungen und Vorteilen von Kameraeinrichtungen und von Verfahren zum Herstellen oben verwiesen.

Das Beleuchtungslicht kann vollständig moduliert sein bzw. ausschließlich modulierte spektrale Anteile aufweisen. Alternativ kann das Beleuchtungslicht zeitlich modulierte und unmodulierte Anteile aufweisen. Zeitlich moduliert ist insbesondere jener Wellenlängenbereich oder ein Teil jenes Wellenlängenbereichs, für den der erste Bildsensor empfindlich ist. Das Beleuchtungslicht kann in einem Wellenlängenbereich, in dem der erste Bildsensor nicht empfindlich ist, unmoduliert sein. Soweit eine Verschlechterung des Signal-Rausch-Verhältnisses in Kauf genommen wird, können auch Anteile des Beleuchtungslichts, für die der erste Bildsensor empfindlich ist, unmoduliert sein. Beispielsweise kann, wenn der erste Bildsensor nur Licht im roten Wellenlängenbereich erfasst, das Beleuchtungslicht zeitlich moduliertes Weißlicht oder gleichzeitig unmoduliertes blaues und grünes Licht und zeitlich moduliertes rotes Licht oder abwechselnd unmoduliertes Weißlicht und zeitlich moduliertes Rotlicht umfassen. Im letztgenannten Fall kann das Abstands-Bildsignal insbesondere in den Zeitintervallen erzeugt werden, in denen das Beleuchtungslicht lediglich zeitlich moduliertes Rotlicht umfasst. Alternativ kann das Beleuchtungslicht gleichzeitig zeitlich unmoduliertes Weißlicht und zeitlich moduliertes Rotlicht umfassen, wodurch das Signal-RauschVerhältnis verschlechtert sein kann.

Das Abstands-Bildsignal wird insbesondere unter Verwendung von Information über die Phase und/oder der Frequenz der periodischen Modulation erzeugt.

Zusätzlich kann ein drittes Intensitäts-Bildsignal, das auf einen dritten vorbestimmten Wellenlängenbereich bezogen ist, mittels des zweiten Bildsensors oder mittels eines dritten Bildsensors erfasst werden, wobei das Farb-Bildsignal unter Verwendung auch des dritten Intensitäts-Bildsignals erzeugt wird.

Bei einem Verfahren zum Erfassen von optischen Eigenschaften und von Raumstruktureigenschaften wird das Farb-Bildsignal insbesondere ohne Verwendung des Abstands-Bildsignals erzeugt.

Bei einem Verfahren zum Erfassen von optischen Eigenschaften und von Raumstruktureigenschaften, wie es hier beschrieben ist, wird ferner insbesondere unter Verwendung des Farb-Bildsignals und des Abstands-Bildsignals ein Stereo-Bildsignal erzeugt.

Mittels des Stereo-Bildsignals kann eine Darstellung eines stereoskopischen Bilds gesteuert werden, beispielsweise durch Darstellung zweier verschiedenfarbiger Bilder eines Anaglyphenbilds, mittels eines Shutter-Verfahrens, eines Polarisationssystems oder eines Linsenrasters (Autostereoskopie).

Bei einem Verfahren zum Erfassen von optischen Eigenschaften und von Raumstruktureigenschaften, wie es hier beschrieben ist, und bei dem die Auflösungen des ersten Intensitäts-Bildsignals und des zweiten Intensitäts-Bildsignals sich voneinander unterscheiden, werden ferner insbesondere die Auflösungen aneinander angepasst.

Das Anpassen der Auflösungen erfolgt insbesondere durch Anpassung der niedrigeren Auflösung an die höhere Auflösung.

Bei einem Verfahren zum Erfassen von optischen Eigenschaften und von Raumstruktureigenschaften, wie es hier beschrieben ist, umfasst das Anpassen der Auflösungen insbesondere ein Interpolieren des Intensitäts-Bildsignals mit der geringeren Auflösung für Positionen von Bildelementen des Bildsignals mit der höheren Auflösung.

Bei einem Verfahren, zum Erfassen von optischen Eigenschaften und von Raumstruktureigenschaften, wie es hier beschrieben ist, umfasst das Anpassen der Auflösungen ein Interpolieren des Intensitäts-Bildsignals mit der geringeren Auflösung für Positionen von Bildelementen des Bildsignals mit der höheren Auflösung unter Verwendung von Information aus dem Bildsignal mit der höheren Auflösung.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einer Kameraeinrichtung;
- Figur 2: eine schematische Darstellung einer Kameraeinrichtung;
- Figur 3: eine schematische Darstellung von Wellenlängenbereichen, Intensitäten und spektralen Empfindlichkeiten;
- Figur 4: eine schematische Darstellung einer zeitlichen Modulation von Beleuchtungslicht;
- Figur 5: eine schematische Darstellung von Wellenlängenbereichen, Intensitäten und spektralen Empfindlichkeiten;
- Figur 6: eine schematische Darstellung einer Signalverarbeitung;
- Figur 7: eine schematische Darstellung einer weiteren Kameraeinrichtung;
- Figur 8: eine schematische Darstellung einer weiteren Kameraeinrichtung;
- Figur 9: schematische Schnittdarstellungen einer Zelle eines Bildsensors;
- Figur 10: schematische Schnittdarstellungen einer Zelle eines weiteren Bildsensors;
- Figur 11: schematische Schnittdarstellungen einer Zelle eines weiteren Bildsensors;
- Figur 12: schematische Schnittdarstellungen einer Zelle eines weiteren Bildsensors;
- Figur 13: schematische Schnittdarstellungen einer Zelle eines weiteren Bildsensors;
- Figur 14: schematische Schnittdarstellungen einer Zelle eines weiteren Bildsensors;
- Figur 15: ein schematisches Flussdiagramm eines Verfahrens zum Herstellen einer Kameraeinrichtung;
- Figur 16: ein schematisches Flussdiagramm eines Verfahrens zum Erfassen von optischen Eigenschaften und von Raumstruktureigenschaften.

### Beschreibung der Ausführungsformen

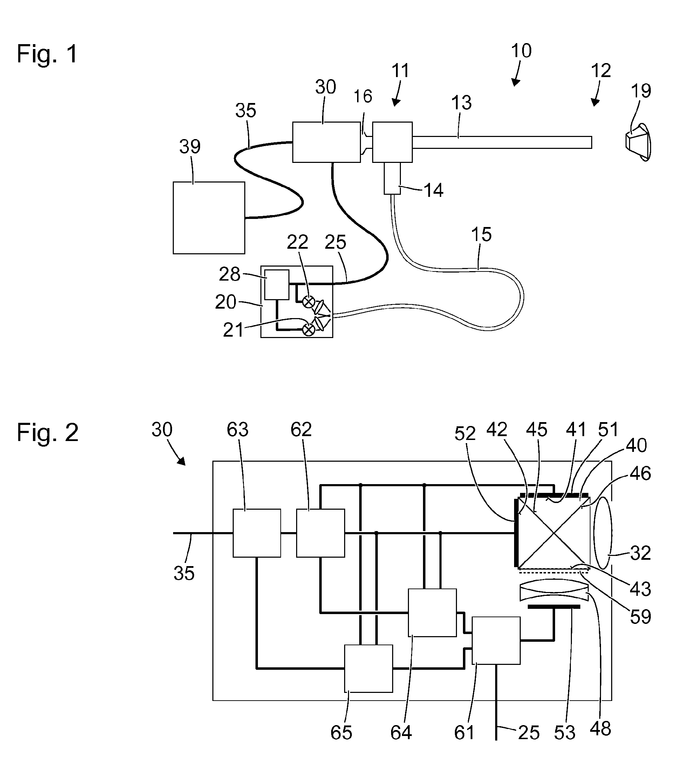

Figur 1 zeigt eine schematische Darstellung eines Endoskops 10 mit einer Lichtquelleneinrichtung 20 und einer Kamera 30. Das Endoskop 10 umfasst ein proximales Ende 11 und ein distales Ende 12. Ein starrer und gerader Schaft 13 erstreckt sich vom proximalen Ende 11 bis zum distalen Ende 12 des Endoskops 10. Der Schaft 13 kann abweichend von der Darstellung in Figur 1 gekrümmt und/oder teilweise oder vollständig flexibel ausgebildet sein. Am proximalen Ende 11 des Endoskops 10 sind eine Kupplung 14 für ein Lichtleitkabel 15 für Beleuchtungslicht und ein Okular 16 vorgesehen.

Die Lichtquelleneinrichtung 20 umfasst eine erste Lichtquelle 21 und eine zweite Lichtquelle 22, deren Licht in das Lichtleitkabel 15 eingekoppelt wird, in Figur 1 angedeutet durch zwei Linsen ohne Bezugszeichen. Ferner umfasst die Lichtquelleneinrichtung 20 eine Steuerung 28 zum Steuern der ersten Lichtquelle 21 und der zweiten Lichtquelle 22.

Eine Kamera 30 ist mit dem Okular 16 des Endoskops 10 mechanisch und optisch gekoppelt. Alternativ kann die Kamera 30 abweichend von der Darstellung in Figur 1 teilweise oder vollständig in das Endoskop 10 integriert sein. Die Kamera 30 ist über ein Signalkabel 25 mit der Lichtquelleneinrichtung 20, insbesondere mit der Steuerung 28 der Lichtquelleneinrichtung 20 gekoppelt. Ferner ist die Kamera 30 über ein weiteres Signalkabel 35 mit einem Bildschirm 39 gekoppelt.

Die Lichtquelleneinrichtung 20 ist ausgebildet, um Beleuchtungslicht zu erzeugen, das mittels des Lichtleitkabels 15 zum proximalen Ende 11 des Endoskops 10 und mittels eines oder mehrerer Lichtleiter im Schaft 13 zum distalen Ende 12 des Endoskops 10 geleitet wird. Am distalen Ende 12 des Endoskops 10 tritt das von der Lichtquelleneinrichtung 20 erzeugte Beleuchtungslicht aus und fällt auf einen Gegenstand bzw. ein Objekt 19.

Vom Objekt 19 reflektiertes oder remittiertes Beleuchtungslicht und vom Objekt 19 (beispielsweise durch Fluoreszenz) emittiertes Licht wird teilweise am distalen Ende 12 in das Endoskop 10 eingekoppelt. Wenn die Kamera 30, wie in Figur 1 dargestellt am proximalen Ende 11 des Endoskops 10 angeordnet ist, wird das vom Objekt 19 ausgehende Licht beispielsweise mittels einer Mehrzahl hintereinander angeordneter Stablinsen im starren Schaft 13 oder, insbesondere im Fall einer elastischen oder plastischen Flexibilität des Schafts 13, mittels eines geordneten Bündels von Lichtleitfasern vom distalen Ende 12 zum proximalen Ende 11 des Endoskops 10 übertragen.

In der Kamera 30 wird das vom Objekt 19 ausgehende Licht auf mehrere lichtempfindliche Bildsensoren geleitet. Dabei entstehen Bilder des Objekts 19 in mehreren verschiedenen Wellenlängenbereichen auf den Bildsensoren. Die Bildsensoren erzeugen Sensorsignale, die von Signalverarbeitungseinrichtungen verarbeitet werden, um ein Stereo-Bildsignal zu erzeugen. Das Stereo-Bildsignal wird mittels des Signalkabels 35 zum Bildschirm 39 übertragen und steuert dort die Darstellungen von zwei Bildern, von denen je eines für das linke und das rechte Auge des Betrachters vorgesehen ist.

Die beiden Bilder werden beispielsweise abwechselnd auf dem Bildschirm 39 dargestellt, wobei der Betrachter eine Shutter-Brille trägt, die synchron zur alternierenden Darstellung der beiden Bilder das linke und dass rechte Auge des Betrachters beschattet. Alternativ werden die beiden Bilder am Bildschirm 39 in Licht mit unterschiedlicher Polarisation dargestellt, wobei der Betrachter zwei unterschiedliche Polarisationsfilter vor seinen Augen trägt. Alternativ können die beiden Bilder am Bildschirm 39 in unterschiedlichen Farben dargestellt sein, wobei der Betrachter zwei verschiedenfarbige Filter vor seinen Augen trägt. Alternativ weist der Bildschirm 39 eine Anordnung von Linsen oder Blenden auf, so dass die beiden Augen des Betrachters teilweise oder vollständig disjunkte Teilmengen von Bildelementen bzw. Pixeln sehen. Alternativ kann der Betrachter zwei Bildschirme, vor jedem Auge einen, tragen. In diesem Fall ist abweichend von der Darstellung in Figur 1 kein Bildschirm 39 erforderlich. Stattdessen wird das Stereo-Bildsignal mittels des Signalkabels 35 oder mittels einer Funkstrecke zu einer brillenähnlichen Einrichtung mit den beiden Bildschirmen am Betrachter übertragen.

Die Lichtquellen 21, 22 der Lichtquelleneinrichtung 20 sind ausgebildet, um Licht mit zwei unterschiedlichen Emissionsspektren zu erzeugen. Insbesondere sind die erste Lichtquelle 21 zum Erzeugen von Licht mit einem ersten Emissionsspektrum und die zweite Lichtquelle 22 zum Erzeugen von Licht mit einem zweiten Emissionsspektrum ausgebildet. Die beiden Emissionsspektren der Lichtquellen 21, 22 können teilweise oder vollständig überlappen oder weitgehend oder vollständig disjunkt sein.

Die Steuerung 28 der Lichtquelleneinrichtung 20 ist ausgebildet, um ein hochfrequentes Modulationssignal zu erzeugen. Das hochfrequente Modulationssignal steuert einerseits die zweite Lichtquelle 22, um die Emission der zweiten Lichtquelle 22 entsprechend zu modulieren, und wird andererseits mittels des Signalkabels 25 zur Kamera 30 übertragen. Alternativ wird abweichend von der Darstellung in Figur 1 lediglich ein Signal, das die Phase der Modulation der Emission der zweiten Lichtquelle 22 anzeigt bzw. in einer vorbestimmten Phasenrelation zu der Modulation steht, zur Kamera übertragen.

Figur 2 zeigt eine schematische Darstellung der Kamera 30 aus Figur 1. Die Kamera 30 umfasst eine abbildende Einrichtung 32, insbesondere ein Objektiv, ein Linsen- oder Spiegelsystem, zur optischen Kopplung der Kamera mit dem Okular 16 des Endoskops 10 (vgl. Figur 1). Einrichtungen zur mechanischen Kopplung der Kamera 30 mit dem Endoskop 10 sind in Figur 2 nicht dargestellt.

Die Kamera 30 umfasst ferner einen Strahlteiler in Gestalt eines Prismas 40 mit Lichtaustrittsflächen 41, 42, 43 und dichroitisch reflektierenden Schichten 45, 46 im Prisma 40. Die dichroitisch reflektierenden Schichten 45, 46 im Prisma 40 sind so ausgebildet, dass durch die abbildende Einrichtung 32 in das Prisma 40 eintretendes Licht abhängig von seiner Wellenlänge an einer der drei Lichtaustrittsflächen 41, 42, 43 verlässt. Insbesondere gelangt Licht mit mittleren Wellenlängen zur zweiten Lichtaustrittsfläche 42, die erste dichroitisch reflektierende Schicht 45 lenkt Licht mit kurzen Wellenlängen zur ersten Lichtaustrittsfläche 41, und die zweite dichroitisch reflektierende Schicht 46 lenkt Licht mit langen Wellenlängen zur dritten Lichtaustrittsfläche 43.

Im Folgenden wird beispielhaft davon ausgegangen, dass die erste dichroitisch reflektierende Schicht 45 Licht im vom menschlichen Auge als blau wahrgenommenen Wellenlängenbereich zur ersten Lichtaustrittsfläche 41 reflektiert, die zweite dichroitisch reflektierende Schicht 46 Licht im vom menschlichen Auge als rot wahrgenommenen Wellenlängenbereich zur dritten Lichtaustrittsfläche 43 reflektiert und Licht im vom menschlichen Auge als grün wahrgenommenen Wellenlängenbereich zur zweiten Lichtaustrittsfläche 42 gelangt. Abweichend von diesem nachfolgend verwendeten Beispiel können jedoch andere reflektierende Eigenschaften der dichroitisch reflektierenden Schichten 45, 46 und entsprechend eine Zuordnung der Lichtaustrittsflächen 41, 42, 43 des Prismas 40 zu anderen Wellenlängenbereichen vorgesehen sein.

An der ersten Lichtaustrittsfläche 41 ist ein dem blauen Wellenlängenbereich zugeordneter Bildsensor 51 angeordnet. An der zweiten Lichtaustrittsfläche 42 ist ein dem grünen Wellenlängenbereich zugeordneter Bildsensor 52 angeordnet. An der dritten Lichtaustrittsfläche 43 kann ursprünglich bzw. vor dem in Figur 2 dargestellten Zustand ein dem roten Wellenlängenbereich zugeordneter Sensor 59 angeordnet gewesen sein. Dieser Bildsensor 59 ist in Figur 2 lediglich in gestrichelter Linie angedeutet, da er entfernt und durch eine Anpassungsoptik 48, beispielsweise einen Anamorphoten, und eine dem roten und infraroten Wellenlängenbereich zugeordneten Bildsensor 53 ersetzt ist. Alternativ kann die hier anhand der Figur 2 dargestellte Konfiguration die ursprüngliche herstellte Konfiguration sein.

Bei dem in Figur 2 dargestellten Beispiel weisen der dem blauen Wellenlängenbereich zugeordnete Bildsensor 51 und der dem grünen Wellenlängenbereich zugeordnete Bildsensor 52 einerseits und der dem roten Wellenlängenbereich zugeordnete Bildsensor 53 andererseits unterschiedliche Größen, d. h. unterschiedliche Breiten und/oder unterschiedliche Höhen auf. Der Anamorphot 48 ist vorgesehen und ausgebildet, um mit dem dem roten Wellenlängenbereich zugeordneten Bildsensor 53 ein Bild zu erfassen, dessen Breite und Höhe den durch die anderen beiden Bildsensoren 51, 52 erfassten Bildern möglichst genau entspricht.

Wenn der dem roten Wellenlängenbereich zugeordnete Bildsensor 53 das gleiche Format aufweist wie die anderen beiden Bildsensoren, kann eine Anpassungsoptik, insbesondere ein Anamorphot 48, entfallen. In diesem Fall ist der dem roten Wellenlängenbereich zugeordnete Bildsensor 53 abweichend von der Darstellung in Figur 2 insbesondere ebenso unmittelbar an der dritten Lichtaustrittsfläche 43 des Prismas 40 angeordnet (insbesondere mit diesem verkittet) wie die anderen Bildsensoren 51, 52 an den anderen beiden Lichtaustrittsflächen 41, 42.

Unabhängig von möglicherweise unterschiedlichen Abmessungen wird nachfolgend davon ausgegangen, dass der dem roten Wellenlängenbereich zugeordnete Bildsensor 53 auch eine andere Auflösung bzw. eine andere, insbesondere geringere Anzahl von Bildelementen bzw. Pixeln aufweist.

Der dem roten und infraroten Wellenlängenbereich zugeordnete Bildsensor 53 ist ein phasenempfindlicher ToF-Bildsensor, der aufgrund einer zeitlich hochfrequent modulierbaren Empfindlichkeit zur Erfassung von Abständen aus Laufzeiten von Lichtsignalen geeignet ist. Bei einer weitverbreiteten Bauform eines ToF-Bildsensors werden die in einem fotoempfindlichen Element (beispielsweise einer Fotodiode) eines Bildelements bzw. Pixels erzeugten Ladungsträger hochfrequent alternierend von zwei Speicherelementen (insbesondere Kondensatoren) gesammelt. Im Fall einer hochfrequent modulierten Beleuchtung des erfassten Objekts ist das Verhältnis der in den Speicherelementen gesammelten Ladungen ebenso wie das Verhältnis der an den Speicherelementen anliegenden Spannungen eine einfache Funktion der Phasendifferenz zwischen der Modulation des auf das fotoempfindliche Element fallenden Lichts und der hochfrequent alternierenden Schaltung der Speicherelemente. Diese Phasendifferenz ist wiederum eine einfache Funktion der Summe der Laufzeit des Beleuchtungslichts zum Objekt und der Laufzeit des reflektierten oder remittierten Lichts vom Objekt zum fotoempfindlichen Element und damit eine Funktion des Abstands. Aus dem Verhältnis der Spannungen an den beiden Speicherelementen eines Bildelements kann deshalb auf den Abstand des auf das Bildelement abgebildeten Oberflächenbereichs eines erfassten Objekts vom Bildsensor geschlossen werden.

Aufgrund des deutlich komplexeren Aufbaus des einzelnen Bildelements bzw. Pixels und insbesondere auch aufgrund der erforderlichen großen Kapazitäten der jedem Bildelement zugeordneten Speicherelemente ist das einzelne Speicherelement eines ToF-Bildsensors in der Regel wesentlich größer als ein Bildelement eines anderen Bildsensors. Entsprechend ist die Auflösung bzw. die Anzahl der Bildelemente eines ToF-Bildsensors in der Regel wesentlich geringer als die Auflösung eines anderen Bildsensors. ToF-Bildsensoren sind in vielen Fällen nicht oder nicht nur im roten Wellenlängenbereich, sondern stattdessen oder zusätzlich im infraroten Wellenlängenbereich empfindlich und werden im infraroten Wellenlängenbereich betrieben.

Nachfolgend wird davon ausgegangen, dass der für den roten Wellenlängenbereich vorgesehene Bildsensor 53 ein ToF-Bildsensor ist, der im roten und im infraroten Wellenlängenbereich empfindlich ist und eine deutlich geringere Auflösung aufweist als die dem blauen und dem grünen Wellenlängenbereich zugeordneten Bildsensoren 51, 52.

Die Kamera 30 umfasst eine erste Signalverarbeitungseinrichtung 61 mit einem Signaleingang, der mit dem Signalausgang des ToF-Bildsensors 53 gekoppelt ist, um von diesem ein Sensorsignal zu empfangen. Die erste Signalverarbeitungseinrichtung 61 ist ausgebildet, um über das Signalkabel 25 von der Lichtquelleneinrichtung 20 (vgl. Figur 1) ein Signal zu erhalten, das zumindest die Phase der Modulation des von der Lichtquelleneinrichtung 20 erzeugten Beleuchtungslichts angibt. Die erste Signalverarbeitungseinrichtung 61 ist ausgebildet, um unter Verwendung des vom ToF-Bildsensor 53 empfangenen Sensorsignals und der Information über die Phase der Modulation des Beleuchtungslichts ein Intensitäts-Bildsignal und ein Abstands-Bildsignal zu erzeugen.

Das von der ersten Signalverarbeitungseinrichtung 61 erzeugte Intensitäts-Bildsignal gibt für jedes Bildelement des ToF-Bildsensors 53 die über mehrere Perioden der Modulation integrierte bzw. summierte Intensität oder Helligkeit an. Das von der ersten Signalverarbeitungseinrichtung 61 erzeugte Intensitäts-Bildsignal ist aufgrund der Eigenschaften des Prismas 40 und der Anordnung des ToF-Bildsensors 53 am Prisma 40 auf den an der dritten Lichtaustrittsfläche 43 des Prismas 40 austretenden roten und infraroten Wellenlängenbereich bezogen.

Das von der ersten Signalverarbeitungseinrichtung 61 erzeugte Abstands-Bildsignal gibt für jedes Bildelement des ToF-Bildsensors 53 den - beispielsweise auf die oben skizzierte Weise ermittelten-Abstand des auf das Bildelement abgebildeten Oberflächenbereichs des Objekts 19 (vgl. Figur 1) von der Kamera 30 oder vom distalen Ende 12 des Endoskops 10 an.

Die erste Signalverarbeitungseinrichtung 61 kann - abweichend von der schematischen Darstellung in Figur 2 - mit dem ToF-Bildsensor 53 integriert sein. Bei dem dargestellten Beispiel ist die erste Signalverarbeitungseinrichtung 61 ausgebildet, um das Intensitäts-Bildsignal einerseits und das Abstands-Bildsignal andererseits an zwei verschiedene nachgeordnete Einrichtungen (und insbesondere über zwei separate Ausgänge) auszugeben.

Die Kamera 30 umfasst eine zweite Signalverarbeitungseinrichtung 62, deren Signaleingänge mit dem Signalausgang des dem blauen Wellenlängenbereich zugeordneten Bildsensors 51, mit dem Signalausgang des dem grünen Wellenlängenbereich zugeordneten Bildsensors 52 und (mittelbar über eine nachfolgend beschriebene Signalverarbeitungseinrichtungen 64) mit einem Signalausgang der ersten Signalverarbeitungseinrichtung 61 gekoppelt sind. Die zweite Signalverarbeitungseinrichtung 62 erhält von den Bildsensoren 51, 52 bzw. von der ersten Signalverarbeitungseinrichtung 61 drei IntensitätsBildsignale, von denen je eines auf den blauen Wellenlängenbereich, eines auf den grünen Wellenlängenbereich und eines auf den roten und infraroten Wellenlängenbereich bezogen ist. Die zweite Signalverarbeitungseinrichtung 62 ist ausgebildet, um aus den Intensitäts-Bildsignalen ein Farb-Bildsignal zu erzeugen. Dieses Farb-Bildsignal enthält - von der mit zunehmendem Abstand aufgrund abnehmender Beleuchtungsstärke abnehmenden Helligkeit und aufgrund unterschiedlicher Abstände unterschiedlicher Schärfe abgesehen - keine Information über Abstände. Insbesondere geht in die Erzeugung des Farb-Bildsignals durch die zweite Signalverarbeitungseinrichtung 62 das von der ersten Signalverarbeitungseinrichtung 61 erzeugte Abstands-Bildsignal nicht ein.

Die Kamera 30 umfasst ferner eine dritte Signalverarbeitungseinrichtung 63 mit Signaleingängen, die mit einem Signalausgang der zweiten Signalverarbeitungseinrichtung 62 und (mittelbar über eine nachfolgend beschriebene Signalverarbeitungseinrichtung 65) mit der ersten Signalverarbeitungseinrichtung 61 gekoppelt sind, um von diesen das Farb-Bildsignal und das Abstands-Bildsignal zu empfangen. Die dritte Signalverarbeitungseinrichtung 63 ist ausgebildet, um unter Verwendung des von der zweiten Signalverarbeitungseinrichtung empfangenen Farb-Bildsignals und des von der ersten Signalverarbeitungseinrichtung empfangenen Abstands-Bildsignals ein Stereo-Bildsignal zu erzeugen, das insbesondere mittels des Signalkabels 35 zum Bildschirm 39 (vgl. Figur 1) übertragen wird.

Die dritte Signalverarbeitungseinrichtung 63 ist ausgebildet, um zwei leicht unterschiedliche, für je ein Auge eines menschlichen Betrachters vorgesehene Bilder zu erzeugen, bei denen Bildelemente bzw. Bildpunkte um vom zugehörigen Abstand abhängige Distanzen in entgegengesetzte Richtungen verschoben sind. Bei Betrachtung zweier durch dieses Stereo-Bildsignal erzeugten Bilder mit je einem Auge entsteht beim menschlichen Betrachter ein dreidimensionaler Bildeindruck, der die Beurteilung von Abständen, Längen, Flächen und Volumina vereinfacht.

Ferner kann die dritte Signalverarbeitungseinrichtung 63 ausgebildet sein, um unter Verwendung des Farb-Bildsignals und des Abstands-Bildsignals Längen, Flächen und Volumina von erfassten Objekten zu berechnen und in einer oder mehreren Tabellen abzulegen oder alphanumerisch optisch oder akustisch auszugeben

Die Kamera 30 umfasst ferner eine vierte Signalverarbeitungseinrichtung 64 zwischen der ersten Signalverarbeitungseinrichtung 61 und der zweiten Signalverarbeitungseinrichtung 62. Die vierte Signalverarbeitungseinrichtung 64 ist vorgesehen und ausgebildet, um das von der ersten Signalverarbeitungseinrichtung 61 erzeugte Intensitäts-Bildsignal zu empfangen und in modifizierter Form an die zweite Signalverarbeitungseinrichtung 62 weiterzugeben.

Die vierte Signalverarbeitungseinrichtung 64 ist ausgebildet, um die Auflösung, d. h. die Anzahl der Spalten und Zeilen der Bildelemente, des von der ersten Signalverarbeitungseinrichtung 61 erzeugten Intensitäts-Bildsignals im roten Bereich an die Auflösungen der Intensitäts-Bildsignale anzupassen, die die zweite Signalverarbeitungseinrichtung 62 von den dem blauen und dem grünen Wellenlängenbereich zugeordneten Bildsensoren 51, 52 empfängt. Insbesondere ist die vierte Signalverarbeitungseinrichtung 64 ausgebildet, um das von der ersten Signalverarbeitungseinrichtung 61 erzeugte Intensitäts-Bildsignal für Positionen von Bildelementen der Intensitäts-Bildsignale, die von den Bildsensoren 51, 52, die dem blauen und dem grünen Wellenlängenbereich zugeordnet sind, herrühren, zu interpolieren.

Die vierte Signalverarbeitungseinrichtung 64 empfängt dazu insbesondere auch die von den Bildsensoren 51, 52, die dem blauen und dem grünen Wellenlängenbereich zugeordnet sind, herrührenden Intensitäts-Bildsignale. Die vierte Signalverarbeitungseinrichtung 64 ist insbesondere ausgebildet, um in den dem blauen und dem grünen Wellenlängenbereich zugeordneten Intensitäts-Bildsignalen Kanten und andere Strukturen zu erkennen und auf das dem roten Wellenlängenbereich zugeordnete Intensitäts-Bildsignal zu übertragen. Insbesondere werden Splines bzw. Polynome oder andere Funktionen an die dem blauen und dem grünen Wellenlängenbereich zugeordneten Intensitäts-Bildsignale angepasst bzw. gefittet und die so gewonnen Koeffizienten bzw. Parameter teilweise oder vollständig bei der Interpolation des dem roten Wellenlängenbereich zugeordneten Intensitäts-Bildsignals verwendet.

Ferner umfasst die Kamera 30 eine fünfte Signalverarbeitungseinrichtung 65 zwischen der ersten Signalverarbeitungseinrichtung 61 und der dritten Signalverarbeitungseinrichtung 63. Die fünfte Signalverarbeitungseinrichtung 65 ist ausgebildet, um das von der ersten Signalverarbeitungseinrichtung 61 erzeugte Abstands-Bildsignal zu empfangen und seine Auflösung an die dem blauen und dem grünen Wellenlängenbereich zugeordneten Intensitäts-Bildsignale anzupassen. Dazu ist die fünfte Signalverarbeitungseinrichtung 65 insbesondere ähnlich oder identisch der vierten Signalverarbeitungseinrichtung 64 ausgebildet.

Die Signalverarbeitungseinrichtungen 61, 62, 63, 64 können in Form diskreter oder integrierter aber separater Schaltungen, insbesondere elektronischer Schaltungen ausgebildet sein. Alternativ können die Signalverarbeitungseinrichtungen 61, 62, 63, 64, 65 jeweils teilweise oder vollständig mit einem der Bildsensoren 51, 52, 53, insbesondere mit dem ToF-Bildsensor 53, oder untereinander integriert sein. Insbesondere ist die erste Signalverarbeitungseinrichtung 61 mit dem ToF-Bildsensor 53 integriert. Die Signalverarbeitungseinrichtungen 61, 62, 63, 64, 65 können jeweils teilweise oder vollständig in Form eines oder mehrerer FPGAs mit vorbestimmter Konfiguration und jeweils teilweise oder vollständig in Form von Software oder Firmware für einen oder mehrere Prozessoren oder einen Computer ausgebildet sein.

Alle Signalverarbeitungseinrichtungen 61, 62, 63, 64, 65 können - wie in Figur 2 angedeutet - zusammen mit dem Strahlteiler und den Bildsensoren 51, 52, 53 in einem gemeinsamen Gehäuse und somit als Teil einer einzigen Kameraeinheit angeordnet sein. Alternativ können diese Signalverarbeitungseinrichtungen 61, 62, 63, 64, 65 teilweise oder vollständig in einer oder mehreren separaten Einheiten angeordnet sein, beispielsweise in Gestalt eines Computers und/oder mit dem Bildschirm 39 (vgl. Figur 1) integriert. Unabhängig von ihrer räumlichen Anordnung, insbesondere unabhängig davon, ob alle Signalverarbeitungseinrichtungen 61, 62, 63, 64, 65 zusammen mit dem Strahlteiler 40 und den Bildsensoren 51, 52, 53 in einem gemeinsamen Gehäuse oder in mehreren separaten Einheiten angeordnet sind, sind alle in Figur 2 dargestellten Signalverarbeitungseinrichtungen 61, 62, 63, 64, 65, der Strahlteiler 40 und die Bildsensoren 51, 52, 53 Bestandteile einer Kameraeinrichtung im Sinne der vorliegenden Beschreibung.

Figur 3 zeigt eine schematische Darstellung von Wellenlängenbereichen, spektralen Empfindlichkeiten und Intensitäten. Der Abszisse ist die Wellenlängen λ (Lambda) zugeordnet. Der Ordinate sind Intensitäten I und spektrale Empfindlichkeiten A zugeordnet. Dem sehr schematischen Charakter der Darstellung entsprechend sind weder an der Abszisse noch an der Ordinate Einheiten angegeben. Die Formen der wiedergegebenen Kurven, insbesondere Steilheit und Verlauf von Flanken, Plateaus, Gestalt von Maxima etc., sind stark vereinfacht und finden in dieser starken Vereinfachung keine Entsprechung in der Realität. Die Kurven dienen lediglich der Verdeutlichung der nachfolgend dargestellten Merkmale und Eigenschaften.

Die spektrale Empfindlichkeit 71 der S-Zapfen des menschlichen Auges, die spektrale Empfindlichkeit 72 der M-Zapfen des menschlichen Auges und die spektrale Empfindlichkeit 73 der L-Zapfen des menschlichen Auges weisen Maxima und Flanken bei unterschiedlichen Wellenlängen auf, überlappen einander jedoch teilweise. Die spektralen Empfindlichkeiten 71, 72, 73 der S-, M- und L-Zapfen des menschlichen Auges definieren den für das menschliche Auge sichtbaren Wellenlängenbereich 80, der sich von ca. 380 nm bis 750 nm erstreckt. Der Wellenlängenbereich, in dem die Empfindlichkeit der S-Zapfen dominiert, wird als blauer Wellenlängenbereich 81 bezeichnet. Der Wellenlängenbereich, in dem die Empfindlichkeit der M-Zapfen dominiert, wird als grüner Wellenlängenbereich 82 bezeichnet. Der Wellenlängenbereich, in dem die Empfindlichkeit der L-Zapfen dominiert, wird als roter Wellenlängenbereich 83 bezeichnet. Zu längeren Wellenlängen hin schließt sich an den roten Wellenlängenbereich 83 der infrarote Wellenlängenbereich 84 außerhalb des für das menschliche Auge sichtbaren Wellenlängenbereichs 80 an.

Bei der Entwicklung von Kameras wird in der Regel darauf geachtet, dass die spektralen Empfindlichkeiten der Kamera in den Farbkanälen weitgehend den spektralen Empfindlichkeiten 71, 72, 73 der S-, Mund L-Zapfen des menschlichen Auges entsprechen. Bei der oben anhand der Figur 2 dargestellten Kamera 30 sind die spektralen Empfindlichkeiten in den Farbkanälen vor allem durch die wellenlängenabhängigen Reflexionseigenschaften der dichroitisch reflektierenden Schichten 45, 46 und durch spektrale Empfindlichkeiten der Bildsensoren 51, 52, 53 bestimmt. ToF-Sensoren weisen typischerweise eine spektrale Empfindlichkeit auf, die sich weit in den infraroten Wellenlängenbereich 84 hinein erstreckt.

Nachfolgend wird davon ausgegangen, dass auch bei langen Wellenlängen die abbildende Einrichtung 32, das Prisma 40 und der Anamorphot 48 transparent und die zweite dichroitisch reflektierende Schicht 46 reflektierend sind. In diesem Fall entspricht die spektrale Empfindlichkeit 74 des dem ToF-Bildsensor 53 zugeordneten Farbkanals der spektralen Empfindlichkeit 74 des ToF-Bildsensors 53. Für den blauen Farbkanal (Bildsensor 51) und den grünen Farbkanal (Bildsensor 52) wird im Folgenden vereinfachend angenommen, dass die spektralen Empfindlichkeiten den spektralen Empfindlichkeiten 71, 72 der S- bzw. M-Zapfen des menschlichen Auges entsprechen.

In Figur 3 sind ferner ein Emissionsspektrum 91 der ersten Lichtquelle 21 (vgl. Figur 1) und ein Emissionsspektrum 92 der zweiten Lichtquelle 22 angedeutet. Das Emissionsspektrum 91 der ersten Lichtquelle 21 umfasst im Wesentlichen den blauen und den grünen Wellenlängenbereich 81, 82. Das Emissionsspektrum 92 der zweiten Lichtquelle 22 umfasst im Wesentlichen den roten Wellenlängenbereich 83. Alternativ kann die zweite Lichtquelle 22 ein alternatives Emissionsspektrum 93 erzeugen, das einen Teil des infraroten Wellenlängenbereichs 84 umfasst.

Figur 4 zeigt eine schematische Darstellung der zeitabhängigen Modulation des von der Lichtquelleneinrichtung 20 (vgl. Figur 1) erzeugten Beleuchtungslichts. In zwei schematischen Diagrammen übereinander sind unten die zeitliche Modulation der Emission der ersten Lichtquelle 21 und oben die zeitliche Modulation der Emission der zweiten Lichtquelle 22 dargestellt. Der Abszisse ist jeweils die Zeit t zuge-ordnet, der Ordinate die Intensität l₂₁ des von der ersten Lichtquelle 21 erzeugten und in das Lichtleitkabel 15 eingekoppelten Beleuchtungslichts bzw. die Intensität l₂₂ der Intensität des von der zweiten Lichtquelle 22 erzeugten und in das Lichtleitkabel 15 eingekoppelten Beleuchtungslichts.

Beleuchtungslicht wird alternierend von der ersten Lichtquelle 21 und der zweiten Lichtquelle 22 erzeugt. In Zeitintervallen, in denen Beleuchtungslicht von der ersten Lichtquelle 21 erzeugt wird, ist die Intensität l₂₁ des von der ersten Lichtquelle 21 erzeugten Beleuchtungslichts im Wesentlichen konstant. In Zeitintervallen, in denen die zweite Lichtquelle 22 Beleuchtungslicht erzeugt, ist die Intensität l₂₂ des von der zweiten Lichtquelle 22 erzeugten Beleuchtungslichts zeitlich hochfrequent moduliert. Die Modulationsfrequenz liegt insbesondere im Bereich von einigen MHz bis einigen GHz.

Die Zeitintervalle, in denen die erste Lichtquelle 21 Beleuchtungslicht erzeugt, und die Zeitintervalle, in denen die zweite Lichtquelle 22 Beleuchtungslicht erzeugt, alternieren insbesondere mit der Bildwiederholfrequenz, die typischerweise zwischen 25 Hz und 100 Hz liegt.

Figur 5 zeigt eine weitere schematische Darstellung von Wellenlängenbereichen, spektralen Empfindlichkeiten und Intensitäten. Die Art der Darstellung entspricht der Darstellung in Figur 3.

Die Wellenlängenbereiche 80, 81, 82, 83, 84 und die spektralen Empfindlichkeiten 71, 72, 73, 74 der Zapfen des menschlichen Auges und des ToF-Bildsensor zugeordneten Farbkanals entsprechen den oben anhand der Figur 3 dargestellten. Ferner ist das bereits in Figur 3 dargestellte alternative Emissionsspektrum 93 der zweiten Lichtquelle 22 dargestellt. Ferner sind in Figur 5 ein alternatives Emissionsspektrum 94 der ersten Lichtquelle 21 (vgl. Figur 1) und ein weiteres alternatives Emissionsspektrum 95 der zweiten Lichtquelle 22 dargestellt. Das alternative Emissionsspektrum 95 der zweiten Lichtquelle 22 ist schmalbandig und entspricht beispielsweise dem Emissionsspektrum eines Halbleiterlasers oder einer schmalbandig emittierenden Halbleiterdiode.

Das alternative Emissionsspektrum 94 der ersten Lichtquelle 21 umfasst neben dem blauen Wellenlängenbereich 81 und dem grünen Wellenlängenbereich 82 auch den roten Wellenlängenbereich 83. Im Fall einer Verwendung einer ersten Lichtquelle 21 mit dem alternativen Emissionsspektrum 94 kann der ToF-Sensor 53 in den Zeitintervallen, in denen die erste Lichtquelle 21 das alternative Emissionsspektrum 94 emittiert wie ein einfacher Bildsensor zum Erfassen eines Intensitäts-Bildsignals für den roten Wellenlängenbereich 83 verwendet werden. In den Zeitintervallen, in denen die zweite Lichtquelle 22 das Emissionsspektrum 92, das alternative Emissionsspektrum 93 oder das alternative Emissionsspektrum 95 erzeugt, kann mittels des ToF-Bildsensors 53 ein Abstands-Bildsignal gewonnen werden.

Wenn die erste Lichtquelle 21 das oben anhand der Figur 3 dargestellte Emissionsspektrum 91 erzeugt, das kein oder nur wenig rotes Licht umfasst, kann in den Zeitintervallen, in denen die zweite Lichtquelle 22 das Emissionsspektrum 92 oder das alternative Emissionsspektrum 93 erzeugt, mittels des ToF-Bildsensors 53 neben dem Abstands-Bildsignal ein auf den roten Farbkanal bezogenen Intensitäts-Bildsignal gewonnen werden.

Wenn sowohl die erste Lichtquelle 21 rotes Licht in nennenswerter Menge erzeugt, beispielsweise in Form des alternativen Emissionsspektrums 94 aus Figur 5, als auch die zweite Lichtquelle 22, können mittels des ToF-Bildsensors 53 in den Zeitintervallen, in denen die erste Lichtquelle 21 Beleuchtungslicht erzeugt, ein erstes Intensitäts-Bildsignal und in den Zeitintervallen, in denen nur die zweite Lichtquelle 22 Beleuchtungslicht erzeugt, ein zweites Intensitäts-Bildsignal erzeugt werden. Beide mittels des ToF-Bildsensors 53 erzeugte Intensitäts-Bildsignale können aufgrund der auch im roten und infraroten Wellenlängenbereich 83, 84 unterschiedlichen Spektren der beiden Lichtquellen 21, 22 zur Verbesserung der Farbwiedergabe im roten Farbkanal verwendet werden. Insbesondere wird eine Linearkombination bzw. eine gewichtete Summe oder Differenz beider mittels des ToF-Bildsensors 53 erzeugten Intensitäts-Bildsignale verwendet, um ein verbessertes Intensitäts-Bildsignal zu erhalten.

Soweit der Überlapp zwischen der spektralen Empfindlichkeit 74 des ToF-Bildsensors 53 und dem Emissionsspektrum (insbesondere dem in Figur 3 gezeigten Emissionsspektrum 91) der ersten Lichtquelle 21 gering ist, kann die erste Lichtquelle 21 abweichend von der Darstellung in Figur 4 ständig betrieben werden. Ein größerer Überlapp zwischen dem Emissionsspektrum 91 der ersten Lichtquelle 21 und der spektralen Empfindlichkeit 74 des ToF-Bildsensors 53 verschlechtert das Signal-Rausch-Verhältnis im mittels des ToF-Bildsensors 53 gewonnen Abstands-Bildsignal.

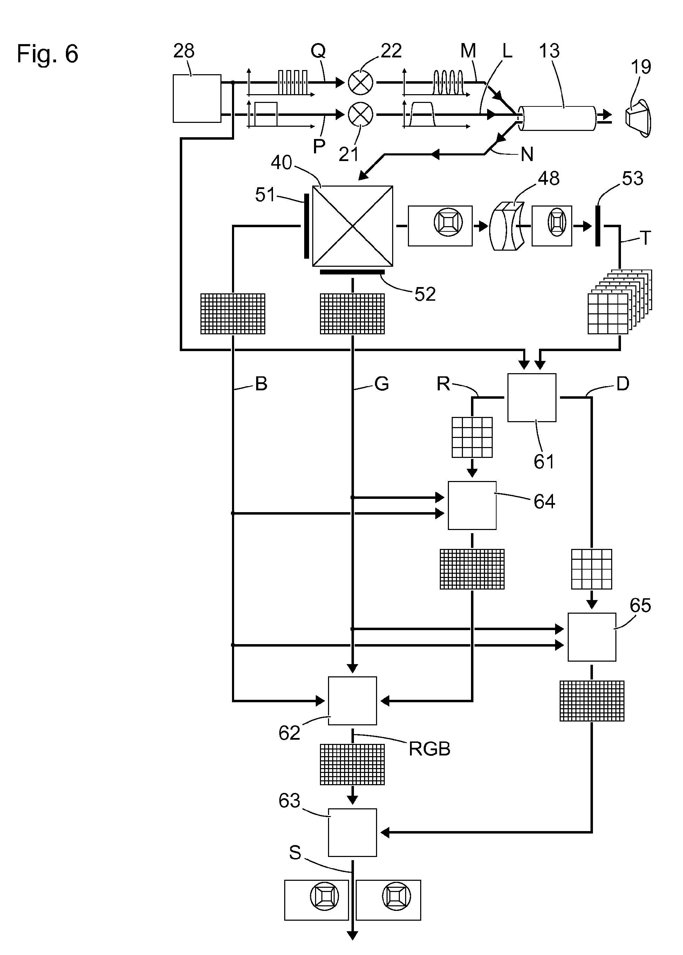

Figur 6 zeigt eine schematische Darstellung einer Signalverarbeitung mittels der Lichtquelleneinrichtung 20, des Endoskops 10 und der Kamera 30 aus den Figuren 1 und 2. Soweit Elemente, Merkmale und Eigenschaften der Lichtquelleneinrichtung, des Endoskops und der Kamera in Figur 6 dargestellt sind, dient dies lediglich der Darstellung der Signalwege. Die räumliche Anordnung in Figur 6 steht in keinem Zusammenhang mit der räumlichen Anordnung in den Figuren 1 und 2 und mit einer realen räumlichen Anordnung.

Die Steuerung 28 der Lichtquelleneinrichtung 20 erzeugt ein Steuersignal P für die erste Lichtquelle 21 und ein Steuersignal Q für die zweite Lichtquelle 22. Die erste Lichtquelle 21 erzeugt entsprechend dem ersten Steuersignal P Beleuchtungslicht L, das insbesondere wie in Figur 4 zeitlich moduliert ist und beispielsweise das Emissionsspektrum 91 aus Figur 3 oder das Emissionsspektrum 94 aus Figur 5 aufweist. Die zweite Lichtquelle 22 erzeugt entsprechend dem zweiten Steuersignal Q Beleuchtungslicht M, das insbesondere wie in Figur 4 oben dargestellt, zeitlich hochfrequent moduliert ist und beispielsweise eines der in den Figuren 3 und 5 dargestellten Emissionsspektren 92, 93, 95 aufweist.

Sowohl das von der ersten Lichtquelle 21 erzeugte Beleuchtungslicht L als auch das von der zweiten Lichtquelle 22 erzeugte Beleuchtungslicht M wird mittels des Lichtleitkabels 15 (vgl. Figur 1) zum proximalen Ende 11 des Endoskops 10 übertragen und beispielsweise mittels einer oder mehrerer Lichtleitfasern im Schaft 13 zum distalen Ende 12 des Endoskops übertragen. Für das Beleuchtungslicht L der ersten Lichtquelle 21 und für das Beleuchtungslicht M der zweiten Lichtquelle 22 können im Lichtleitkabel 15 und im Schaft 13 des Endoskops 10 separate Lichtleiter vorgesehen sein. Alternativ werden sowohl das von der ersten Lichtquelle 21 erzeugte Beleuchtungslicht L als auch das von der zweiten Lichtquelle 22 erzeugte Beleuchtungslicht M mittels desselben Lichtleiters oder mittels derselben Lichtleiter übertragen.

Am distalen Ende 12 des Endoskops 10 (vgl. Figur 1) tritt das Beleuchtungslicht L, M aus und fällt auf das zu betrachtende Objekt 19. Das Beleuchtungslicht L, M wird vom Objekt 19 reflektiert oder remittiert. Ein Teil N des vom Objekt 19 reflektierten oder remittierenden Lichts tritt am distalen Ende 12 in den Schaft 13 des Endoskops 10 ein und wird beispielsweise mittels einer Serie von Stablinsen oder mittels eines geordneten Bündels von Lichtleitern zum Okular 16 des Endoskops 10 und weiter zum Prisma 40 der Kamera 30 (vgl. Figur 2) übertragen.

Im blauen Wellenlängenbereich 81 (vgl. Figur 3) liegende Anteile des Lichts N gelangen zu dem dem blauen Wellenlängenbereich 81 zugeordneten Bildsensor 51 und erzeugen dort ein Bild des Objekts 19 in blauem Licht. Der dem blauen Wellenlängenbereich 81 zugeordnete Bildsensor 51 erzeugt ein dem blauen Wellenlängenbereich 81 zugeordnetes Intensitäts-Bildsignal B oder ein Sensorsignal, das das Intensitäts-Bildsignal B umfasst.

Im grünen Wellenlängenbereich 82 (vgl. Figur 3) liegende Anteile des Lichts N gelangen zu dem dem grünen Wellenlängenbereich 82 zugeordneten Bildsensor 52 und erzeugen dort ein Bild des Objekts 19 in grünem Licht. Der dem grünen Wellenlängenbereich 82 zugeordnete Bildsensor 52 erzeugt ein dem grünen Wellenlängenbereich 82 zugeordnetes Intensitäts-Bildsignal G oder ein Sensorsignal, das das Intensitäts-Bildsignal G umfasst.

Im roten Wellenlängenbereich 83 und/oder im infraroten Wellenlängenbereich 84 liegende Anteile des Lichts N vom Objekt 19 gelangen über einen Anamorphoten 48 zum ToF-Bildsensor 53 und erzeugen dort ein Bild des Objekts 19 in rotem und/oder infrarotem Licht. Zwischen dem Prisma 40 und dem Anamorphoten 48 und zwischen dem Anamorphoten 48 und dem ToF-Bildsensor 53 sind jeweils Bilder des Objekts 19 mit unterschiedlichen Abmessungen angedeutet. Die unterschiedlichen Abmessungen dieser beiden Bilder deuten schematisch die Wirkung des Anamorphoten 48 und die Anpassung der Abmessungen des Bilds an die Abmessungen des ToF-Bildsensors 53 an.

Der ToF-Bildsensor 53 erzeugt ein Sensorsignal T, das sowohl Information über Intensitäten bzw. Helligkeiten im roten Wellenlängenbereich als auch über Abstände enthält.

In den Signalpfaden des auf den blauen Wellenlängenbereich bezogenen Intensitäts-Bildsignals B, des auf den grünen Wellenlängenbereich bezogenen Intensitäts-Bildsignals G und des Sensorsignals T sind mittels karierter Rechtecke unterschiedliche Formate der Intensitäts-Bildsignale B, G und des Sensorsignals T angedeutet. Entsprechend der hier zugrunde gelegten deutlich geringeren Auflösung bzw. Anzahl von Bildelementen des ToF-Bildsensors 53 ist das Sensorsignal T auf eine wesentlich geringere Anzahl von Spalten und Zeilen von Bildelementen bezogen als die Intensitäts-Bildsignale B, G. Beispielsweise erzeugen die dem blauen und dem grünen Farbkanal zugeordneten Bildsensoren 51, 52 jeweils Bildsignale, die Bilder mit einer Auflösung von 1920 x 1080 oder 1920 x 1200 Bildelementen bzw. Pixeln (Full-HD) wiedergeben. Das Sensorsignal T ist entsprechend der Auflösung des ToF-Bildsensors 53 beispielsweise auf ca. 200 x 200 Bildelemente bezogen.

In der symbolischen Darstellung des Sensorsignals T in Figur 6 sind mehrere Ebenen angedeutet. Dies entspricht der Tatsache, dass das Sensorsignal T neben der Zweidimensionalität des enthaltenen bzw. repräsentierten Intensitäts-Bilds Information über eine dritte Dimension, nämlich Abstände abgebildeter Oberflächenelemente vom ToF-Bildsensor 53 bzw. vom distalen Ende 12 des Endoskops 10, enthält.

Das Sensorsignal T des ToF-Bildsensors 53 gelangt zur ersten Signalverarbeitungseinrichtung 61. Die erste Signalverarbeitungseinrichtung 61 empfängt ferner das Steuersignal Q für die zweite Lichtquelle 22 oder ein entsprechendes Signal, das Information über die Phase des Steuersignals und des von der zweiten Lichtquelle 22 erzeugten Beleuchtungslichts M enthält. Unter Verwendung des Sensorsignals T des ToF-Bildsensors 53 und des Steuersignals Q der Steuerung 28 erzeugt die erste Signalverarbeitungseinrichtung 61 ein Intensitäts-Bildsignal R und ein Abstands-Bildsignal D. Das Intensitäts-Bildsignal R ist auf den roten Wellenlängenbereich 83 und/oder auf den infraroten Wellenlängenbereich 84 bezogen.

Insbesondere ist das Intensitäts-Bildsignal R nur auf den roten Wellenlängenbereich 83 bezogen. Im Fall des Emissionsspektrums 94 (vgl. Figur 5) der ersten Lichtquelle 21 wird das Intensitäts-Bildsignal R insbesondere ausschließlich aufgrund von Photonen erzeugt, die während des Zeitintervalls auf den ToF-Bildsensor 53 fallen, in dem die erste Lichtquelle 21 Beleuchtungslicht L emittiert. Alternativ (insbesondere im Fall des in Figur 3 dargestellten Emissionsspektrums 91 der ersten Lichtquelle 21) oder zusätzlich kann das Intensitäts-Bildsignal R oder ein weiteres Intensitäts-Bildsignal aufgrund von Photonen erzeugt werden, die während der Zeitintervalle, in denen die zweite Lichtquelle 22 Licht emittiert, im ToF-Bildsensor 53 erzeugt werden.

Das Intensitäts-Bildsignal R enthält - von einer aufgrund abnehmender Beleuchtungsstärke mit zunehmendem Abstand abnehmenden Helligkeit abgesehen - keine Information über den Abstand des Objekts 19 vom ToF-Bildsensor 53 (entlang des optischen Pfads) oder über den Abstand des Objekts 19 vom distalen Ende 12 des Endoskops 10. Die Information über Abstände ist lediglich im Abstands-Bildsignal D enthalten.

Die erste Signalverarbeitungseinrichtung 61 erzeugt das Intensitäts-Bildsignal R und das Abstands-Bildsignal D mit der Auflösung des ToF-Bildsensors 53 und des Sensorsignals T. Die Auflösung des Intensitäts-Bildsignals R wird durch die vierte Signalverarbeitungseinrichtung 64 erhöht und an die Auflösung der Intensitäts-Bildsignale B, G zu den blauen und grünen Wellenlängenbereichen angepasst. Die zweite Signalverarbeitungseinrichtung 62 erzeugt unter Verwendung der Intensitäts-Bildsignale B, G zum blauen und zum grünen Wellenlängenbereich und des in seiner Auflösung angepassten Intensitäts-Bildsignals R zum roten Wellenlängenbereich ein Farb-Bildsignal RGB.

Die fünfte Signalverarbeitungseinrichtung 65 erhöht die Auflösung des Abstands-Bildsignals D und passt sie an die Auflösung der Intensitäts-Bildsignale B, G und des Farb-Bildsignals RGB an. Die dritte Signalverarbeitungseinrichtung 63 erzeugt unter Verwendung des Farb-Bildsignals RGB und des in seiner Auflösung angepassten Abstands-Bildsignals D ein Stereo-Bildsignal S, das zwei Bilder enthält bzw. wiedergibt, eines für jedes Auge eines Betrachters.

Bei der hier beschriebenen Ausführungsform der Kamera 30 kann der Signalpfad vom ToF-Bildsensor 53 zur zweiten Signalverarbeitungseinrichtung 62 bzw. die Verwendung des aus dem Sensorsignal T des ToF-Bildsensors 53 erzeugten Intensitäts-Bildsignals R für die Erzeugung eines Farb-Bildsignals RGB einen besonders vorteilhaften Aspekt darstellen. Insbesondere ermöglicht dieser Signalpfad gegenüber vielen anderen herkömmlichen Ansätzen die Verwendung von lediglich drei Bildsensoren 51, 52, 53. Insbesondere kann zur Herstellung der Kamera 30 ein herkömmlicher Strahlteiler bzw. ein herkömmliches Prisma 40 mit drei dem blauen, dem grünen und dem roten Farbkanal zugeordneten Bildsensoren 51, 52, 53 verwendet werden. Ausgehend von diesem herkömmlichen Strahlteiler wird ein Bildsensor 59 (vgl. Figur 2) - insbesondere der dem roten Farbkanal zugeordnete Bildsensor - entfernt und durch einen ToF-Bildsensor ersetzt.

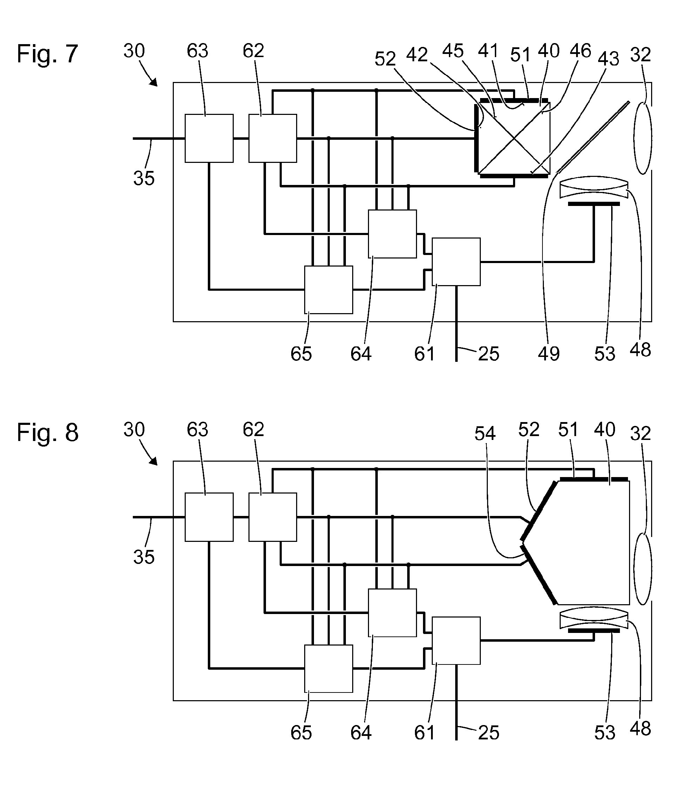

Figur 7 zeigt eine schematische Darstellung einer weiteren Ausführungsform einer Kamera 30, die in einigen Merkmalen und Eigenschaften der oben anhand der Figuren 1 bis 6 dargestellten Kamera ähnelt. Nachfolgend sind lediglich Merkmale, Eigenschaften und Funktionen der Kamera 30 beschrieben, in denen sich diese von der oben anhand der Figuren 1 bis 6 dargestellten unterscheidet.

Die in Figur 7 dargestellte Kamera 30 unterscheidet sich von der oben anhand der Figuren 1 bis 6 dargestellten Kamera insbesondere dadurch, dass insgesamt vier Bildsensoren vorgesehen sind. Einem Strahlteiler in Form eines Prismas 40 ähnlich dem Prisma der oben anhand der Figuren 1 bis 6 dargestellten Kamera ist ein weiterer Strahlteiler 49 in Gestalt eines teildurchlässigen Spiegels vorgeschaltet, der zwischen der abbildenden Einrichtung 32 und dem Prisma 40 angeordnet ist. Am Prisma 40 sind drei Bildsensoren 51, 52, 54 angeordnet, die dem blauen Wellenlängenbereich 81, dem grünen Wellenlängenbereich 82 bzw. dem roten Wellenlängenbereich 83 zugeordnet sind. Der weitere Strahlteiler 49 ist vorgesehen und ausgebildet, um Licht im infraroten Wellenlängenbereich 84 und optional Licht eines Teils des roten Wellenlängenbereichs 83 über einen Anamorphoten 48 auf einen ToF-Bildsensor 53 zu lenken.

Die erste Signalverarbeitungseinrichtung 61 ist ausgebildet, um ähnlich wie bei der oben anhand der Figuren 1 bis 6 dargestellten Kamera unter Verwendung des Sensorsignals T des ToF-Bildsensors 53 und des Steuersignals Q für die zweite Lichtquelle 22 (vgl. Figur 6) ein Intensitäts-Bildsignal und ein Abstands-Bildsignal zu erzeugen. Die zweite Signalverarbeitungseinrichtung 62 ist ausgebildet, um unter Verwendung der Intensitäts-Bildsignale, die von den am Prisma 40 angeordneten Bildsensoren 51, 52, 54 erzeugt werden, und des von der ersten Signalverarbeitungseinrichtung 61 erzeugten Intensitäts-Bildsignals ein Farb-Bildsignal zu erzeugen. Durch die Verwendung des vom ToF-Bildsensor 53 herrührenden Intensitäts-Bildsignals zur Erzeugung des Farb-Bildsignals kann eine Verbesserung der Farbwiedergabe insbesondere im roten Farbkanal, erzielt werden. Insbesondere kann eine Linearkombination bzw. eine gewichtete Summe oder Differenz des mittels des ToF-Bildsensors 53 erzeugten Intensitäts-Bildsignals und des mittels des dem roten Wellenlängenbereich 83 zugeordneten Bildsensors 54 verwendet werden, um ein verbessertes Intensitäts-Bildsignal für den roten Farbkanal zu erhalten.

Figur 8 zeigt eine schematische Darstellung einer weiteren Kamera, die in einigen Merkmalen, Eigenschaften und Funktionen den oben anhand der Figuren 1 bis 7 dargestellten Kameras ähnelt. Nachfolgend sind lediglich Merkmale, Eigenschaften und Funktionen dargestellt, in denen die Kamera 30 sich von den oben anhand der Figuren 1 bis 7 dargestellten Kameras unterscheidet.

Die in Figur 8 dargestellte Kamera 30 unterscheidet sich von der oben anhand der Figur 7 dargestellten Kamera insbesondere dadurch, dass anstelle eines Prismas und eines weiteren Strahlteilers ein Prisma 40 mit vier Lichtaustrittsflächen als Strahlteiler vorgesehen ist. Dichroitisch reflektierende Schichten im Prisma 40 sind in Figur 8 der Einfachheit halber nicht dargestellt.

Sowohl die anhand der Figur 7 dargestellte Anordnung zweier Strahlteiler in Serie als auch die anhand der Figur 8 dargestellte Verwendung eines Strahlteilers mit vier (oder mehr) Lichtaustrittsflächen und die Verwendung einer entsprechenden Anzahl von Bildsensoren vergrößern die Masse und das Bauvolumen. Der Massenzuwachs der Kamera kann zumindest teilweise durch Leichtbau kompensiert werden, beispielsweise durch ein Gehäuse aus tiefgezogenem und geschweißtem korrosionsbeständigen Titanblech.

Anstelle eines Strahlteilers mit drei oder mehr Bildsensoren kann eine Kamera abweichend von der Darstellung anhand der Figuren 2 bis 8 lediglich einen ToF-Bildsensor und einen weiteren Bildsensor umfassen. Der weitere Bildsensor ist beispielsweise ein Bayer-Sensor oder ein anderer Bildsensor mit zwei oder mehr Gruppen von Bildelementen, vor denen Filter in zwei (oder mehr) verschiedenen Farben angeordnet sind. Der weitere Bildsensor erzeugt ein Sensorsignal, das eine entsprechende Anzahl von Intensitäts-Bildsignale enthält oder in eine entsprechende Anzahl von Intensitäts-Bildsignalen umgewandelt werden kann.

Es wird eine Linearkombination bzw. eine gewichtete Summe oder Differenz aus dem vom ToF-Bildsensor herrührenden Intensitäts-Bildsignal und einem der von dem weiteren Bildsensor herrührenden Intensitäts-Bildsignale gebildet. Ferner können weitere Linearkombinationen aus dem vom ToF-Bildsensor herrührenden Intensitäts-Bildsignal und je einem der von dem weiteren Bildsensor herrührenden Intensitäts-Bildsignale gebildet werden. Diese Linearkombination oder Linearkombinationen können einer Verbesserung der Farbwiedergabe bewirken und/oder beispielsweise eine Unterscheidung von benignem und malignem Gewebe vereinfachen.

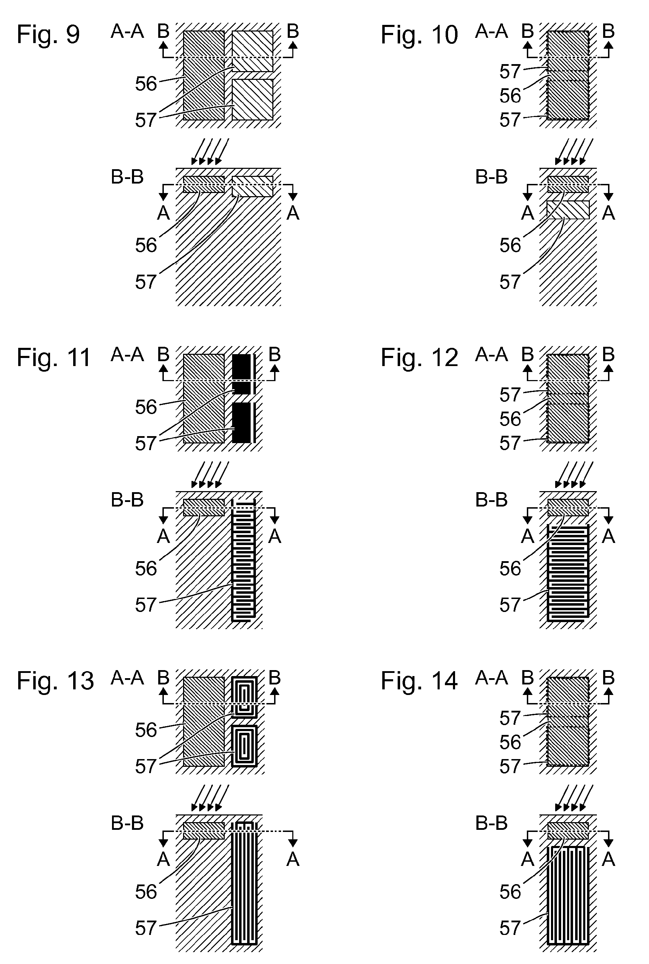

Figur 9 zeigt schematische Schnittdarstellungen einer Zelle bzw. eines Bildelements bzw. eines Pixels eines ToF-Bildsensors. Die oben dargestellte Schnittebene A-A ist parallel zur Oberfläche des ToF-Bildsensors bzw. zu der Bildfläche, in die das zu erfassende Bild optisch abgebildet wird. Die in Figur 9 unten dargestellte Schnittebene B-B ist senkrecht zur Schnittebene A-A und senkrecht zur Bildfläche. Die Lage der Schnittfläche A-A ist im Schnitt entlang der Ebene B-B dargestellt, die Lage der Schnittebene B-B ist im Schnitt entlang der Ebene A-A dargestellt.

In Figur 9 sind ein fotoempfindliches Element 56 und zwei Speicherelemente 57 (insbesondere Kondensatoren) dargestellt. Jedes Bildelement enthält weitere Bauteile, insbesondere Transistoren oder andere Schalter und Leitungen, die in Figur 9 nicht dargestellt sind, weil sie wesentlich weniger Bauraum beanspruchen. Der von den Speicherelementen 57 eingenommene Bauraum liegt typischerweise in einer zum Bauraum des fotoempfindlichen Elements 56 vergleichbaren Größenordnung. Bei herkömmlichen ToF-Bildsensoren sind das fotoempfindliche Element 56 und die Speicherelemente 57 im Wesentlichen in einer Ebene nebeneinander angeordnet.

Zur Vergrößerung der Auflösung eines ToF-Bildsensors ist eine Verkleinerung des einzelnen Bildelements erforderlich. Es wird vorgeschlagen, das Verhältnis zwischen der Speicherkapazität der Speicherelemente 57 und der von ihnen eingenommenen Fläche zu erhöhen, um ihren Flächenbedarf und damit die Fläche des einzelnen Bildelements bzw. Pixels zu verringern und die Auflösung des ToF-Bildsensors zu erhöhen. Dazu wird insbesondere vorgeschlagen, anstelle herkömmlicher Kondensatoren mit festem Dielektrikum zwischen den Elektroden Elektrolytkondensatoren zu verwenden. Elektrolytkondensatoren ermöglichen bei gleichem Bauraum eine wesentlich größere Kapazität. Umgekehrt ist bei gleicher Kapazität ein wesentlich kleinerer Bauraum erforderlich. Der Elektrolyt kann durch Aufdrucken regelmäßig angeordneter Tröpfchen-Arrays, Aufdampfen bzw. Kondensieren oder auf andere Weise eingebracht werden. Ferner wird vorgeschlagen mittels porösem Silizium oder anderer poröser Halbleiterstrukturen oder mittels Nano-Kohlenstoffröhrchen die Flächen der Kondensatorelektroden mikroskopisch zu strukturieren und damit zu vergrößern, um das Verhältnis zwischen Kapazität und eingenommenen Bauraum zu verbessern.

Figur 10 zeigt schematische Schnittdarstellungen einer weiteren Ausführungsform eines Bildelements eines ToF-Bildsensors, die in einigen Merkmalen und Eigenschaften der Ausführungsform der Figur 9 ähnelt. Die Art der Darstellung in Figur 10 entspricht der Art der Darstellung in Figur 9.

Das Bildelement in Figur 10 unterscheidet sich von dem oben anhand der Figur 9 dargestellten Bildelement dadurch, dass die Speicherelemente 57 nicht neben, sondern unter dem fotoempfindlichen Element 56 angeordnet sind. Dadurch kann die vom einzelnen Bildelement eingenommene Fläche drastisch reduziert werden, bei dem dargestellten Beispiel auf etwa die Hälfte.

Figur 11 zeigt schematische Schnittdarstellungen einer weiteren Ausführungsform eines Bildelements eines ToF-Bildsensors, die in einigen Merkmalen und Eigenschaften den Ausführungsformen der Figuren 9 und 10 ähnelt. Die Art der Darstellung in Figur 11 entspricht der Art der Darstellung in den Figuren 9 und 10.

Die Ausführungsform der Figur 11 unterscheidet sich von der Ausführungsform der Figur 9 insbesondere dadurch, dass das einzelne Speicherelement 57 eine in größere Tiefe reichende sandwichartige Stapelung mehrerer oder vieler planarer Kondensatorelektroden umfasst, die alternierend mit einem von zwei Polen elektrisch leitfähig verbunden sind. Dies kann eine drastische Erhöhung des Verhältnisses zwischen der wirksamen Kondensatorfläche bzw. der wirksamen Flächen der Kondensatorelektroden einerseits und der parallel zur Schnittebene A-A gemessenen Grundfläche der Speicherelemente 57 andererseits ermöglichen.

Figur 12 zeigt schematische Schnittdarstellungen einer weiteren Ausführungsform eines Bildelements eines ToF-Bildsensors, die in einigen Merkmalen und Eigenschaften den Ausführungsformen der Figuren 9 bis 11 ähnelt. Die Art der Darstellung in Figur 12 entspricht der Art der Darstellung in den Figuren 9 bis 11.

Die Ausführungsform der Figur 12 unterscheidet sich von der Ausführungsform der Figur 11 insbesondere dadurch, dass die Speicherelemente 57 nicht neben, sondern - ähnlich wie bei der Ausführungsform der Figur 10 - unter dem fotosensitiven Element 56 angeordnet sind. Dies kann eine weitere drastische Reduzierung der durch das einzelne Bildelement eingenommenen Fläche (gemessen parallel zur Schnittebene A-A) und eine entsprechende Verbesserung der Auflösung des ToF-Bildsensors ermöglichen.

Figur 13 zeigt schematische Schnittdarstellungen einer weiteren Ausführungsform eines Bildelements eines ToF-Bildsensors, die in einigen Merkmalen und Eigenschaften den Ausführungsformen der Figuren 9 bis 12 ähnelt. Die Art der Darstellung in Figur 13 entspricht der Art der Darstellung in den Figuren 9 bis 12.

Die Ausführungsform von Figur 13 unterscheidet sich von der Ausführungsform der Figur 11 insbesondere dadurch, dass anstelle horizontaler bzw. zur Schnittebene A-A paralleler Kondensatorelektroden vertikale bzw. zur Schnittebene A-A senkrechte Kondensatorelektroden vorgesehen sind. Die Kondensatorelektroden weisen insbesondere die Gestalt von Mantelflächen zylindrischer oder prismatischer Säulen auf. Mehrere Elektrodenflächen unterschiedlicher Querschnitte sind ineinander angeordnet, durch Dielektrikum voneinander isoliert und alternierend mit den beiden Polen eines Speicherelements elektrisch leitfähig verbunden. Anstelle eines rechteckigen Querschnitts kann ein Speicherelement 57 jeweils einen kreisförmigen oder elliptischen Querschnitt in der Ebene A-A aufweisen. Ferner können die Elektroden in einer Doppelspirale angeordnet sein.

Figur 14 zeigt schematische Schnittdarstellungen einer weiteren Ausführungsform eines Bildelements eines ToF-Bildsensors, die in einigen Merkmalen und Eigenschaften den Ausführungsformen der Figuren 9 bis 13 ähnelt. Die Art der Darstellung in Figur 14 entspricht der Art der Darstellung in den Figuren 9 bis 13.

Die Ausführungsform der Figur 14 unterscheidet sich von der oben anhand der Figur 13 dargestellten Ausführungsform insbesondere dadurch, dass die Speicherelemente 57 nicht neben, sondern - ähnlich wie bei den Ausführungsformen der Figuren 10 und 12 - unter dem fotosensitiven Element 56 angeordnet sind.

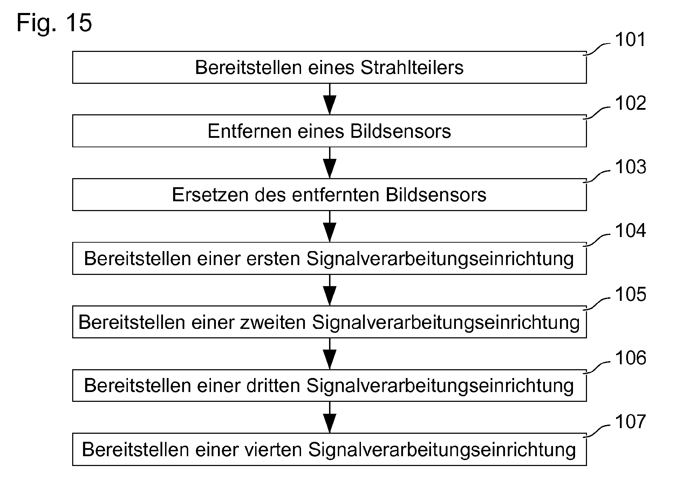

Figur 15 zeigt ein schematisches Flussdiagramm eines Verfahrens zum Herstellen einer Kamera bzw. einer Kameraeinrichtung. Das Verfahren ist auch zur Herstellung einer Kamera oder einer Kameraeinrichtung, deren Eigenschaften und Merkmale von den oben anhand der Figuren 1 bis 8 dargestellten abweichen, geeignet. Trotzdem werden nachfolgend zur Vereinfachung des Verständnisses beispielhaft Bezugszeichen aus den Figuren 1 bis 8 verwendet.

Bei einem ersten Schritt 101 wird ein Strahlteiler 40 mit mehreren Bildsensoren 51, 52, 59 bereitgestellt. Der Strahlteiler 40 ist insbesondere ein Prisma mit mehreren dichroitisch reflektierenden Schichten, die bewirken, dass Licht abhängig von seiner Wellenlänge auf unterschiedliche Bildsensoren 51, 52, 59 abgebildet wird. Nachfolgend wird beispielhaft davon ausgegangen, dass die Bildsensoren 51, 52, 59 am Strahlteiler 40 jeweils einem der Farbkanäle blau, grün und rot zugeordnet sind.

Bei einem zweiten Schritt 102 wird ein Bildsensor 59 vom Strahlteiler 40 entfernt. Nachfolgend wird davon ausgegangen, dass der entfernte Bildsensor 59 dem roten Wellenlängenbereich bzw. dem roten Farbkanal zugeordnet war.

Bei einem dritten Schritt 103 wird der entfernte Bildsensor 59 durch einen aufgrund zeitlich hochfrequent modulierbarer Empfindlichkeit zur ortsaufgelösten Erfassung von Abständen geeigneten Bildsensor 53 ersetzt. Nachfolgend wird davon ausgegangen, dass der ersetzende Bildsensor 53 ein ToF-Bildsensor ist und eine geringere Auflösung aufweist als die am Strahlteiler verbleibenden Bildsensoren 51, 52.

Wenn der ersetzende Bildsensor 53 eine andere Breite oder eine Höhe aufweist als die am Strahlteiler 40 verbleibenden Bildsensoren 51, 52 kann der dritte Schritt 103 des Ersetzens einen Schritt des Bereitstellens einer Anpassungseinrichtung umfassen. Wenn der ersetzende Bildsensor 53 ein anderes Verhältnis zwischen Höhe und Breite aufweist als die am Strahlteiler 40 verbleibenden Bildsensoren 51, 52, ist oder umfasst die Anpassungseinrichtung 48 insbesondere einen Anamorphoten.

Bei einem vierten Schritt 104 wird eine erste Signalverarbeitungseinrichtung bereitgestellt und mit dem ersetzenden ToF-Bildsensor 53 gekoppelt. Die beim vierten Schritt 104 bereitgestellte erste Signalverarbeitungseinrichtung 61 kann mit dem ersetzenden ToF-Bildsensor integriert sein. In diesem Fall fallen der dritte Schritt 103 und der vierte Schritt 104 zusammen. Die erste Signalverarbeitungseinrichtung 61 ist zum Empfangen eines Sensorsignals vom ersetzenden ToF-Bildsensor 53 und zum Empfangen einer Information über die zeitliche Modulation von Beleuchtungslicht (insbesondere über die Phase der zeitlichen Modulation) und zum Erzeugen eines Intensitäts-Bildsignals und eines Abstands-Bildsignals ausgebildet.

Bei einem fünften Schritt 105 wird eine zweite Signalverarbeitungseinrichtung 62 bereitgestellt und mit der ersten Signalverarbeitungseinrichtung 61 und den am Strahlteiler 40 verbleibenden Bildsensoren 51, 52 gekoppelt. Die beim fünften Schritt 105 bereitgestellte zweite Signalverarbeitungseinrichtung 62 ist ausgebildet, um von dem am Strahlteiler 40 verbleibenden Bildsensoren 51, 52 Intensitäts-Bildsignal B, G, die auf den blauen bzw. auf den grünen Wellenlängenbereich bezogen sind, und von der ersten Signalverarbeitungseinrichtung 61 ein Intensitäts-Bildsignal, das auf den roten Wellenlängenbereich bezogen ist, zu empfangen. Ferner ist die zweite Signalverarbeitungseinrichtung 62 ausgebildet, um unter Verwendung der Intensitäts-Bildsignale ein Farb-Signal RGB zu erzeugen.

Bei einem sechsten Schritt 106 wird eine dritte Signalverarbeitungseinrichtung 63 bereitgestellt und mit der ersten Signalverarbeitungseinrichtung 61 und der zweiten Signalverarbeitungseinrichtung 62 gekoppelt. Die beim sechsten Schritt 106 bereitgestellte dritte Signalverarbeitungseinrichtung 63 ist ausgebildet, um von der ersten Signalverarbeitungseinrichtung 61 das Abstands-Bildsignal und von der zweiten Signalverarbeitungseinrichtung 62 das Farb-Bildsignal zu empfangen und unter Verwendung des Abstands-Bildsignals D und des Farb-Bildsignals RGB ein Stereo-Bildsignal S zu erzeugen.

Bei einem optionalen siebten Schritt 107 wird eine vierte Signalverarbeitungseinrichtung 64 zur Anpassung der Auflösung des von der ersten Signalverarbeitungseinrichtung 61 erzeugten Intensitäts-Bildsignals R an die Auflösung der Intensitäts-Bildsignale B, G von den am Strahlteiler 40 verbliebenen Bildsensoren 51, 52 anzupassen. Der siebte Schritt 107 kann insbesondere dann entfallen, wenn die Auflösung des beim dritten Schritt 103 bereitgestellten ToF-Bildsensors 53 der Auflösung der am Strahlteiler 40 verbliebenen Strahlteiler 51, 52 entspricht.

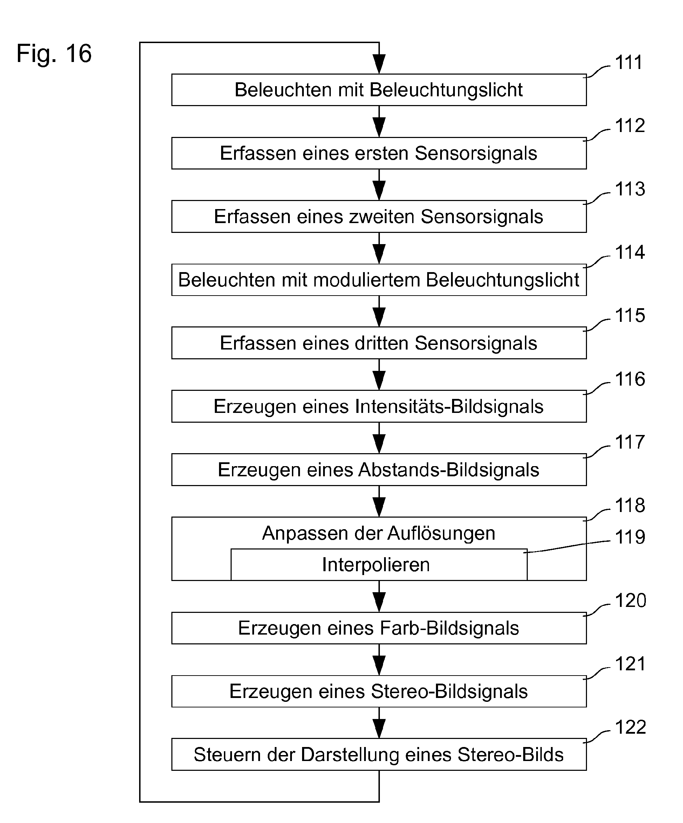

Figur 16 zeigt ein schematisches Flussdiagramm eines Verfahrens zum Erfassen von optischen Eigenschaften in mehreren verschiedenen Wellenlängenbereichen und von Raumstruktureigenschaften eines Objekts. Das Verfahren ist mittels einer Kamera oder einer Kameraeinrichtung ausführbar, die von den oben anhand der Figuren 1 bis 8 dargestellten Merkmalen und Eigenschaften abweichende Merkmale und Eigenschaften aufweist. Ferner ist das Verfahren mit einer Kamera oder Kameraeinrichtung ausführbar, die mittels eines Verfahrens hergestellt ist, das anhand der Figur 15 dargestellten Verfahren abweichende Schritte, Eigenschaften und Merkmale aufweist. Trotzdem werden nachfolgend beispielhaft Bezugszeichen aus den Figuren 1 bis 8 verwendet, um das Verständnis zu vereinfachen.

Bei einem ersten Schritt 111 wird ein Objekt 19 mit Beleuchtungslicht, das von einer ersten Lichtquelle 21 erzeugt ist, beleuchtet. Das Emissionsspektrum der ersten Lichtquelle 21 umfasst insbesondere die vom menschlichen Auge als blau und grün wahrgenommenen Wellenlängenbereiche und optional zusätzlich den vom menschlichen Auge als rot wahrgenommenen Wellenlängenbereich. Bei einem zweiten Schritt 112 wird ein erstes Sensorsignal von einem dem blauen Wellenlängenbereich zugeordneten Bildsensor erfasst. Das erste Sensorsignal umfasst ein dem blauen Wellenlängenbereich bzw. dem blauen Farbkanal zugeordnetes Intensitäts-Bildsignal. Bei einem dritten 113 wird ein zweites Sensorsignal von einem dem grünen Wellenlängenbereich zugeordneten Bildsensor 52 erfasst. Das zweite Sensorsignal umfasst ein dem grünen Wellenlängenbereich zugeordnetes Intensitäts-Bildsignal. Der zweite Schritt 112 und der dritte Schritt 113 werden insbesondere gleichzeitig mit dem ersten Schritt 111 ausgeführt.

Bei einem vierten Schritt 114 wird das Objekt 19 mittels zeitlich hochfrequent modulierten Beleuchtungslichts beleuchtet, das von einer zweiten Lichtquelle 22 erzeugt ist. Die zweite Lichtquelle erzeugt insbesondere ein Emissionsspektrum im roten und/oder infraroten Wellenlängenbereich.

Bei einem fünften Schritt 105 wird ein drittes Sensorsignal eines dem roten und/oder infraroten Wellenlängebereich zugeordneten Bildsensors 53 erfasst. Das dritte Sensorsignal enthält sowohl Information über die zeitlich gemittelte Intensität des vom Objekt 19 reflektierten oder remittierten Beleuchtungslichts (und ggf. ferner des vom Objekt 19 emittierten Lichts) als auch Information über Abstände des Objekts 19 bzw. von dessen Oberflächenbereichen vom Bildsensor, von der Kamera oder von einem distalen Ende 12 eines Endoskops 10.

Bei einem sechsten Schritt 116 wird unter Verwendung des dritten Sensorsignals T ein Intensitäts-Bildsignal R, das auf den roten und/oder auf den infraroten Wellenlängenbereich bezogen ist, erzeugt. Bei einem siebten Schritt 117 wird unter Verwendung des dritten Sensorsignals und einer Information über die Modulation, insbesondere die Phase der Modulation, des Beleuchtungslichts ein Abstands-Bildsignal D erzeugt. Der sechste Schritt 116 und der siebte Schritt 117 werden insbesondere unmittelbar nach dem fünften Schritt 115 von einer ersten Signalverarbeitungseinrichtung 61 ausgeführt.

Bei einem achten Schritt 118 werden die Auflösung des beim zweiten Schritt erfassten ersten Sensorsignals, des beim dritten Schritt erfassten zweiten Sensorsignals und des beim sechsten Schritt erzeugten Intensitäts-Bildsignals aneinander angepasst. Insbesondere wird die niedrigere Auflösung des beim sechsten Schritt erzeugten Intensitäts-Bildsignals R an die höhere Auflösung der im ersten Sensorsignal und dem zweiten Sensorsignal enthaltenen Intensitäts-Bildsignale B, G angepasst. Der achte Schritt 118 enthält insbesondere einen neunten Schritt einer Interpolation des beim sechsten Schritt 116 erzeugten Intensitäts-Bildsignals für Positionen von Bildelementen der von den anderen beiden Intensitäts-Bildsignalen B, G beschriebenen Bilder.

Bei einem zehnten Schritt 120 wird unter Verwendung der Intensitäts-Bildsignale B, G, R ein Farb-Bildsignal RGB erzeugt. Der zehnte Schritt 120 wird insbesondere durch eine zweite Signalverarbeitungseinrichtung 62 ausgeführt.

Bei einem elften Schritt 121 wird unter Verwendung des Farb-Bildsignals RGB und des Abstands-Bildsignals D ein Stereo-Bildsignal S erzeugt. Der elfte Schritt 121 wird insbesondere von einer dritten Signalverarbeitungseinrichtung 63 ausgeführt.

Bei einem optionalen zwölften Schritt 122 wird die Darstellung eines Stereo-Bilds durch das Stereo-Bildsignal S gesteuert. Der zwölfte Schritt 122 wird insbesondere durch einen Bildschirm 39 oder mit Hilfe eines Bildschirms 39 ausgeführt.

Alle dargestellten Schritte können mit einer Bildwiederholfrequenz, die beispielsweise im Bereich von 25 Hz bis 100 Hz liegt, wiederholt werden, um beim Betrachter den Eindruck einer kontinuierlichen Bewegung zu erhalten. Der erste Schritt 111, der zweite Schritt 112 und der dritte Schritt 113 einerseits und der vierte Schritt 114, der fünfte Schritt 115, der sechste Schritt 116, der siebte Schritt 117 und der achte Schritt 118 andererseits bilden zwei Verfahrensteile bzw. Gruppen von Schritten, die abwechselnd bzw. alternierend, gleichzeitig oder teilweise gleichzeitig ausgeführt werden können. Beispielsweise bei der anhand der Figur 4 dargestellten Steuerung von Beleuchtungslicht werden der erste Schritt 111, der zweite Schritt 112 und der dritte Schritt 113 im Wesentlichen innerhalb des Zeitintervalls ausgeführt, innerhalb dessen die erste Lichtquelle 21 (vgl. Figuren 1 und 6) Beleuchtungslicht emittiert. Der vierte Schritt 114, der fünfte Schritt 115, der sechste Schritt 116, der siebte Schritt 117 und der achte Schritt 118 werden innerhalb des Zeitintervalls ausgeführt, innerhalb dessen die zweite Lichtquelle 22 zeitlich hochfrequent moduliertes Beleuchtungslicht emittiert.

### Bezugszeichen

- 10: Endoskop
- 11: proximales Ende des Endoskops 10
- 12: distales Ende des Endoskops 10
- 13: Schaft des Endoskops 10
- 14: Kupplung für Lichtleitkabel für Beleuchtungslicht
- 15: Lichtleitkabel
- 16: Okular
- 19: Objekt bzw. Gegenstand
- 20: Lichtquelleneinrichtung
- 21: erste Lichtquelle der Lichtquelleneinrichtung 20
- 22: zweite Lichtquelle der Lichtquelleneinrichtung 20
- 25: Signalkabel zwischen Lichtquelleneinrichtung 20 und Kamera 30
- 28: Steuerung der Lichtquelleneinrichtung 20 für die Lichtquellen 21, 22
- 30: Kamera
- 32: abbildende Einrichtung
- 35: Signalkabel zum Bildschirm 39
- 39: Bildschirm
- 40: Prisma
- 41: erste Lichtaustrittsfläche des Prismas 40
- 42: zweite Lichtaustrittsfläche des Prismas 40
- 43: dritte Lichtaustrittsfläche des Prismas 40
- 44: vierte Lichtaustrittsfläche des Prismas 40
- 45: erste dichroitisch reflektierende Schicht im Prisma 40
- 46: zweite dichroitisch reflektierende Schicht im Prisma 40
- 48: Anamorphot
- 49: weiterer Strahlteiler
- 51: Bildsensor an der ersten Lichtaustrittsfläche 41 des Prismas 40 für den blauen Wellenlängenbereich 81
- 52: Bildsensor an der zweiten Lichtaustrittsfläche 42 des Prismas 40 für den grünen Wellenlängenbereich 82
- 53: (ToF-)Bildsensor an der dritten Lichtaustrittsfläche 43 des Prismas 40 für den roten und infraroten Wellenlängenbereich
- 54: Bildsensor an einer weiteren Lichtaustrittsfläche des Prismas 40
- 56: photoempfindliches Element eines ToF-Bildsensors 53
- 57: Speicherelement eines ToF-Bildsensors 53
- 59: entfernter Bildsensor
- 61: erste Signalverarbeitungseinrichtung (zum Erzeugen eines Intensitäts-Bildsignals und eines Abstands-Bildsignals)
- 62: zweite Signalverarbeitungseinrichtung (zum Erzeugen eines Farb-Bildsignals)
- 63: dritte Signalverarbeitungseinrichtung (zum Erzeugen eines Stereo-Bildsignals)
- 64: vierte Signalverarbeitungseinrichtung (zum Anpassen der Auflösung)
- 65: fünfte Signalverarbeitungseinrichtung (zum Anpassen der Auflösung)
- 71: spektrale Empfindlichkeit der S-Zapfen des menschlichen Auges und des blauen Farbkanals der Kamera 30
- 72: spektrale Empfindlichkeit der M-Zapfen des menschlichen Auges und des grünen Farbkanals der Kamera 30
- 73: spektrale Empfindlichkeit der L-Zapfen des menschlichen Auges
- 74: spektrale Empfindlichkeit des ToF-Bildsensors 53
- 80: für das menschliche Auge sichtbarer Wellenlängenbereich
- 81: blauer Wellenlängenbereich
- 82: grüner Wellenlängenbereich
- 83: roter Wellenlängenbereich
- 84: infraroter Wellenlängenbereich
- 91: Emissionsspektrum der ersten Lichtquelle 21
- 92: Emissionsspektrum der zweiten Lichtquelle 22
- 93: alternatives Emissionsspektrum der zweiten Lichtquelle 22
- 94: alternatives Emissionsspektrum der ersten Lichtquelle 21
- 95: alternatives Emissionsspektrum der zweiten Lichtquelle 22
- 101: Bereitstellen eines Strahlteilers 40 mit mehreren Bildsensoren 51, 52, 59
- 102: Entfernen eines Bildsensors 59
- 103: Ersetzen des entfernten Bildsensors 59 durch einen ersten Bildsensor 53
- 104: Bereitstellen einer ersten Signalverarbeitungseinrichtung 61
- 105: Bereitstellen einer zweiten Signalverarbeitungseinrichtung 62
- 106: Bereitstellen einer dritten Signalverarbeitungseinrichtung 63
- 107: Bereitstellen einer vierten Signalverarbeitungseinrichtung 64
- 111: erster Schritt (Beleuchten des Objekts mit Beleuchtungslicht L in einem blauen Wellenlängenbereich und in einem grünen Wellenlängenbereich)
- 112: zweiter Schritt (Erfassen eines ersten Sensorsignals, das ein erstes Intensitäts-Bildsignal B umfasst, mittels eines für einen blauen Wellenlängenbereich vorgesehenen Bildsensors 51)
- 113: dritter Schritt (Erfassen eines zweiten Sensorsignals, das ein zweites Intensitäts-Bildsignal G umfasst, mittels eines für einen grünen Wellenlängenbereich vorgesehenen Bildsensors 52)
- 114: vierter Schritt (Beleuchten des Objekts mit zeitlich moduliertem Beleuchtungslicht M)
- 115: fünfter Schritt (Erfassen eines dritten Sensorsignals T mittels eines dritten Bildsensors mit zeitlich hochfrequent modulierter Empfindlichkeit)
- 116: sechster Schritt (Ermitteln eines dritten Intensitäts-Bildsignals R aus dem dritten Sensorsignal T)
- 117: siebter Schritt (Ermitteln eines Abstands-Bildsignals D unter Verwendung von Information über die Modulation des zeitlich modulierten Beleuchtungslichts M und des dritten Sensorsignals T)
- 118: achter Schritt (Anpassen der Auflösungen des ersten Intensitäts-Bildsignals B, des zweiten Intensitäts-Bildsignals G und des dritten Intensitäts-Bildsignals R aneinander)
- 119: neunter Schritt (Interpolieren des dritten Intensitäts-Bildsignals R für Positionen von Bildelementen des ersten Bildsignals B)
- 120: zehnter Schritt (Erzeugen eines Farb-Bildsignals aus dem ersten Intensitäts-Bildsignal B, dem zweiten Intensitäts-Bildsignal G und dem dritten Intensitäts-Bildsignal R)
- 121: elfter Schritt (Erzeugen eines Stereo-Bildsignals S aus dem Farb-Bildsignal RGB und dem Abstands-Bildsignal D)
- 122: zwölfter Schritt (Steuern der Darstellung eines Stereo-Bilds mittels des Stereo-Bildsignals S)

- B: Intensitäts-Bildsignal im blauen Spektralbereich
- D: Abstands-Bildsignal
- G: Intensitäts-Bildsignal im grünen Spektralbereich
- L: weißes oder blau-grünes Beleuchtungslicht
- M: zeitlich moduliertes rotes Beleuchtungslicht
- N: vom Objekt 19 reflektiertes oder remittiertes oder emittiertes Licht
- P: Steuersignal für erste Lichtquelle 21
- Q: Steuersignal für zweite Lichtquelle 22
- R: Intensitäts-Bildsignal im roten Spektralbereich
- RGB: Farb-Bildsignal
- S: Stereo-Bildsignal
- T: Sensorsignal des ersten (ToF-)Bildsensors 51

## Patentansprüche

1. Kameraeinrichtung (30) zur Erfassung von optischen Eigenschaften in mehreren verschiedenen Wellenlängenbereichen (81, 82, 83, 84) und von Raumstruktureigenschaften eines Objekts (19), mit:
einem Strahlteiler (40) zur Auftrennung von Licht, das von einem Objekt (19) kommt, in die mehreren verschiedenen Wellenlängenbereiche (81, 82, 83, 84);
einem ersten Bildsensor (53) zum Erzeugen eines Sensorsignals (T), das auf einen ersten vorbestimmten Wellenlängenbereich (83, 84) der mehreren verschiedenen Wellenlängenbereiche bezogen ist, wobei der erste Bildsensor (53) aufgrund einer zeitlich modulierbaren Empfindlichkeit zur Erfassung eines Abstands eines Objekts (19) von der Kameraeinrichtung (30) aus einer Laufzeit von Licht (L, M, N) ausgebildet und phasenempfindlich ist;
einer ersten Signalverarbeitungseinrichtung (61) zum Erzeugen eines ersten IntensitätsBildsignals (R) und eines Abstands-Bildsignals (D) unter Verwendung von Information über die zeitliche Modulation von Beleuchtungslicht (M) und des ersten Sensorsignals (T), wobei das Abstands-Bildsignal (D) räumliche Struktureigenschaften des Objekts (19) darstellt;
wenigstens einem zweiten Bildsensor (51, 52) zum Erzeugen (10) eines zweiten Sensorsignals, das ein zweites Intensitäts-Bildsignal (B, G) für einen zweiten vorbestimmten Wellenlängenbereich (81, 82) der mehreren verschiedenen Wellenlängenbereiche umfasst;
einer zweiten Signalverarbeitungseinrichtung (62) zum Erzeugen eines Farb-Bildsignals (RGB) aus dem ersten Intensitäts-Bildsignal (R) und dem zweiten Intensitäts-Bildsignal (G, B), wobei das Farb-Bildsignal (RGB) optische Eigenschaften des Objekts (19) darstellt.

2. Kameraeinrichtung (30) nach dem vorangehenden Anspruch, bei der der erste Wellenlängenbereich (83, 84) zumindest teilweise innerhalb des für das menschliche Auge sichtbaren Wellenlängenbereichs (80) liegt.

3. Kameraeinrichtung (30) nach einem der vorangehenden Ansprüche, bei der die zweite Signalverarbeitungseinrichtung (62) ausgebildet ist, um das Farb-Bildsignal (RGB) ohne Verwendung des Abstands-Bildsignals (D) zu erzeugen.

4. Kameraeinrichtung (30) nach einem der vorangehenden Ansprüche, bei der der erste Bildsensor (53) und der zweite Bildsensor (51, 52) unterschiedliche Auflösungen aufweisen, ferner mit:
einer Signalverarbeitungseinrichtung (64) zum Anpassen einer ersten Auflösung des ersten Intensitäts-Bildsignals (R) und einer zweiten Auflösung des zweiten Intensitäts-Bildsignals (G, B) aneinander.

5. Kameraeinrichtung (30) nach dem vorangehenden Anspruch, bei der die weitere Signalverarbeitungseinrichtung (64) ausgebildet ist, um zum Anpassen verschiedener Auflösungen das Intensitäts-Bildsignal (R) mit der geringeren Auflösung für Positionen von Bildelementen des Intensitäts-Bildsignals (G, B) mit der höheren Auflösung zu interpolieren.

6. Kameraeinrichtung (30) nach dem vorangehenden Anspruch, bei der die weitere Signalverarbeitungseinrichtung (64) ausgebildet ist, um zum Interpolieren Information aus dem Intensitäts-Bildsignal (G, B) mit der höheren Auflösung zu verwenden.

7. Endoskop (10) oder Exoskop mit einer Kameraeinrichtung (30) nach einem der vorangehenden Ansprüche.

8. Verfahren zum Herstellen einer Kameraeinrichtung (30) zur Erfassung von optischen Eigenschaften in mehreren verschiedenen Wellenlängenbereichen (81, 82, 83) und von Raumstruktureigenschaften eines Objekts (19), mit folgenden Schritten:
Bereitstellen (101) eines Strahlteilers (40) zur Auftrennung von Licht in mehrere verschiedene Wellenlängenbereiche (81, 82, 83), wobei an dem Strahlteiler (40) für jeden der mehreren verschiedenen Wellenlängenbereiche (81, 82, 83) je ein lichtempfindlicher Bildsensor (51, 52, 59) vorgesehen ist,
Entfernen (102) eines Bildsensors (59) von dem Strahlteiler (40);
Ersetzen (103) des entfernten Bildsensors (59) durch einen ersten Bildsensor (53), der aufgrund zeitlich modulierbarer Empfindlichkeit zur Erfassung eines Abstands eines Objekts (19) von der Kameraeinrichtung (30) aus einer Laufzeit von Licht (L, M, N) ausgebildet und phasenempfindlich ist,
wobei ein zweiter Bildsensor (51, 52) am Strahlteiler (40) verbleibt,
wobei der erste Bildsensor (53) in dem Wellenlängenbereich (83), der dem entfernten Bildsensor (59) zugeordnet war, empfindlich ist,
ferner mit folgenden Schritten:
Bereitstellen (104) einer ersten Signalverarbeitungseinrichtung (61) zum Empfangen eines ersten Sensorsignals (T) von dem ersten Bildsensor (53) und zum Erzeugen eines ersten Intensitäts-Bildsignals (R) und eines Abstands-Bildsignals (D) unter Verwendung des ersten Sensorsignals (T) des ersten Bildsensors (53),
Bereitstellen einer zweiten Signalverarbeitungseinrichtung (62) zum Empfangen des ersten Intensitäts-Bildsignals (R), zum Empfangen eines zweiten Intensitäts-Bildsignals (B, G) für einen zweiten vorbestimmten Wellenlängenbereich der mehreren verschiedenen Wellenlängenbereiche von dem zweiten Bildsensor (61, 62) und zum Erzeugen eines Farb-Bildsignals (RGB) unter Verwendung des ersten Intensitäts-Bildsignals (R) und des zweiten Intensitäts-Bildsignals (G, B).

9. Verfahren nach Anspruch 8, ferner mit folgendem Schritt:
Bereitstellen (107) einer weiteren Signalverarbeitungseinrichtung (64) zum Anpassen einer ersten Auflösung des ersten Intensitäts-Bildsignals (R) und einer zweiten Auflösung des zweiten Intensitäts-Bildsignals (G, B) aneinander.

10. Verfahren zum Erfassen von optischen Eigenschaften in mehreren verschiedenen Wellenlängenbereichen (81, 82, 83) und von Raumstruktureigenschaften eines Objekts (19), mit folgenden Schritten:
Beleuchten (114) des Objekts (19) mit in vorbestimmter Weise zeitlich moduliertem Beleuchtungslicht (L, M);
Erfassen (115) eines ersten Sensorsignals (T), das auf einen ersten vorbestimmten Wellenlängenbereich (83, 84) der mehreren verschiedenen Wellenlängenbereiche bezogen ist, mittels eines ersten Bildsensors (53) mit zeitlich modulierter Empfindlichkeit, der phasenempfindlich ist;
Erzeugen (116) eines ersten Intensitäts-Bildsignals (R) unter Verwendung des ersten Sensorsignals (T);
Erzeugen (117) eines Abstands-Bildsignals (D) unter Verwendung von Information über die zeitliche Modulation des Beleuchtungslichts (L, M) und des ersten Sensorsignals (T);
Erfassen (113) eines zweiten Intensitäts-Bildsignals (G, B), das auf einen zweiten vorbestimmten Wellenlängenbereich (81, 82) der mehreren verschiedenen Wellenlängenbereiche bezogen ist, mittels eines zweiten Bildsensors (51, 52);
Erzeugen (120) eines Farb-Bildsignals (RGB) unter Verwendung des ersten Intensitäts-Bildsignals (R) und des zweiten Intensitäts-Bildsignals (G, B),
wobei das Farb-Bildsignal (RGB) optische Eigenschaften des Objekts (19) und das Abstands-Bildsignal (R) Raumstruktureigenschaften des Objekts (19) darstellen.

11. Verfahren nach dem vorangehenden Anspruch, bei dem das Farb-Bildsignal (RGB) ohne Verwendung des Abstands-Bildsignals (D) erzeugt (118) wird.

12. Verfahren nach einem der Ansprüche 10 bis 11, bei dem die Auflösungen des ersten Intensitäts-Bildsignals (R) und des zweiten Intensitäts-Bildsignals (G, B) sich voneinander unterscheiden, ferner mit folgendem Schritt:
Anpassen (118) der Auflösungen aneinander.

13. Verfahren nach dem vorangehenden Anspruch, bei dem das Anpassen (118) der Auflösungen ein Interpolieren (119) des Intensitäts-Bildsignals (R) mit der geringeren Auflösung für Positionen von Bildelementen des Bildsignals (G, B) mit der höheren Auflösung unter Verwendung von Information aus dem Bildsignal (G, B) mit der höheren Auflösung umfasst.

## Claims

1. Camera device (30) for acquiring optical properties in a plurality of different wavelength ranges (81, 82, 83, 84) and spatial structure properties of an object (19), comprising:
a beam splitter (40) for splitting light coming from an object (19) into the plurality of different wavelength ranges (81, 82, 83, 84);
a first image sensor (53) for generating a sensor signal (T) related to a first predetermined wavelength range (83, 84) of the plurality of different wavelength ranges, wherein the first image sensor (53), on the basis of a temporally modulatable sensitivity, is designed for acquiring a distance between an object (19) and the camera device (30) from a time of flight of light (L, M, N) and is phase-sensitive;
a first signal processing device (61) for generating a first intensity image signal (R) and a distance image signal (D) using information about the temporal modulation of illumination light (M) and the first sensor signal (T), wherein the distance image signal (D) represents spatial structure properties of the object (19);
at least one second image sensor (51, 52) for generating (10) a second sensor signal, which comprises a second intensity image signal (B, G) for a second predetermined wavelength range (81, 82) of the plurality of different wavelength ranges;
a second signal processing device (62) for generating a color image signal (RGB) from the first intensity image signal (R) and the second intensity image signal (G, B), wherein the color image signal (RGB) represents optical properties of the object (19).

2. Camera device (30) according to the preceding claim, wherein the first wavelength range (83, 84) lies at least partly within the wavelength range (80) visible to the human eye.

3. Camera device (30) according to either of the preceding claims, wherein the second signal processing device (62) is designed to generate the color image signal (RGB) without using the distance image signal (D).

4. Camera device (30) according to any of the preceding claims, wherein the first image sensor (53) and the second image sensor (51, 52) have different resolutions, furthermore comprising:
a signal processing device (64) for matching a first resolution of the first intensity image signal (R) and a second resolution of the second intensity image signal (G, B) to one another.

5. Camera device (30) according to the preceding claim, wherein the further signal processing device (64) is designed to interpolate, for the purpose of matching different resolutions, the intensity image signal (R) having the lower resolution for positions of pixels of the intensity image signal (G, B) having the higher resolution.

6. Camera device (30) according to the preceding claim, wherein the further signal processing device (64) is designed to use, for the purpose of interpolation, information from the intensity image signal (G, B) having the higher resolution.

7. Endoscope (10) or exoscope comprising a camera device (30) according to any of the preceding claims.

8. Method for producing a camera device (30) for acquiring optical properties in a plurality of different wavelength ranges (81, 82, 83) and spatial structure properties of an object (19), comprising the following steps:
providing (101) a beam splitter (40) for splitting light into a plurality of different wavelength ranges (81, 82, 83), wherein a respective light-sensitive image sensor (51, 52, 59) is provided on the beam splitter (40) for each of the plurality of different wavelength ranges (81, 82, 83);
removing (102) an image sensor (59) from the beam splitter (40);
replacing (103) the removed image sensor (59) by a first image sensor (53), which, on the basis of temporally modulatable sensitivity, is designed for acquiring a distance between an object (19) and the camera device (30) from a time of flight of light (L, M, N) and is phase-sensitive,
wherein a second image sensor (51, 52) remains at the beam splitter (40),
wherein the first image sensor (53) is sensitive in the wavelength range (83) which was assigned to the removed image sensor (59),
furthermore comprising the following steps:
providing (104) a first signal processing device (61) for receiving a first sensor signal (T) from the first image sensor (53) and for generating a first intensity image signal (R) and a distance image signal (D) using the first sensor signal (T) of the first image sensor (53),
providing a second signal processing device (62) for receiving the first intensity image signal (R), for receiving a second intensity image signal (B, G) for a second predetermined wavelength range of the plurality of different wavelength ranges from the second image sensor (61, 62) and for generating a color image signal (RGB) using the first intensity image signal (R) and the second intensity image signal (G, B).

9. Method according to Claim 8, furthermore comprising the following step:
providing (107) a further signal processing device (64) for matching a first resolution of the first intensity image signal (R) and a second resolution of the second intensity image signal (G, B) to one another.

10. Method for acquiring optical properties in a plurality of different wavelength ranges (81, 82, 83) and spatial structure properties of an object (19), comprising the following steps:
illuminating (114) the object (19) with illumination light (L, M) that is temporally modulated in a predetermined manner;
acquiring (115) a first sensor signal (T), which is related to a first predetermined wavelength range (83, 84) of the plurality of different wavelength ranges, by means of a first image sensor (53) having temporally modulated sensitivity, which first image sensor is phase-sensitive;
generating (116) a first intensity image signal (R) using the first sensor signal (T);
generating (117) a distance image signal (D) using information about the temporal modulation of the illumination light (L, M) and the first sensor signal (T);
acquiring (113) a second intensity image signal (G, B), which is related to a second predetermined wavelength range (81, 82) of the plurality of different wavelength ranges, by means of a second image sensor (51, 52);
generating (120) a color image signal (RGB) using the first intensity image signal (R) and the second intensity image signal (G, B),
wherein the color image signal (RGB) represents optical properties of the object (19) and the distance image signal (R) represents spatial structure properties of the object (19).

11. Method according to the preceding claim, wherein the color image signal (RGB) is generated (118) without using the distance image signal (D).

12. Method according to any of Claims 10 to 11, wherein the resolutions of the first intensity image signal (R) and of the second intensity image signal (G, B) differ from one another, furthermore comprising the following step:
matching (118) the resolutions to one another.

13. Method according to the preceding claim, wherein matching (118) the resolutions comprises interpolating (119) the intensity image signal (R) having the lower resolution for positions of pixels of the image signal (G, B) having the higher resolution using information from the image signal (G, B) having the higher resolution.

## Revendications

1. Dispositif de caméra (30) destiné à saisir des propriétés optiques dans plusieurs plages différentes (81, 82, 83, 84) de longueurs d'onde et les propriétés structurelles spatiales d'un objet (19), le dispositif présentant :
un diviseur (40) de faisceau qui divise la lumière provenant d'un objet (19) en les différentes plages (81, 82, 83, 84) de longueurs d'onde,
un capteur (53) d'image qui forme un signal (T) de capteur apporté à une première plage (83, 84) de longueurs d'onde prédéterminée parmi les différentes plages de longueurs d'onde, le premier capteur (53) d'image étant configuré sur la base d'une sensibilité modulable dans le temps pour saisir la distance d'un objet (19) par rapport au dispositif de caméra (30) à partir du temps de parcours de la lumière (L, M, N) et étant sensible à la phase,
un premier dispositif (61) de traitement de signal qui forme un premier signal (R) d'intensité d'image et un signal (D) de distance d'image en utilisant des informations concernant la modulation dans le temps de la lumière d'éclairage (M) et du premier signal (T) de capteur, le signal (D) de distance d'image représentant des propriétés structurelles spatiales de l'objet (19),
au moins un deuxième capteur (51, 52) d'image qui forme (10) un deuxième signal de capteur qui comporte un deuxième signal (B, G) d'intensité d'image dans une deuxième plage (81, 82) de longueurs d'onde prédéterminée parmi les différentes plages de longueurs d'onde, et
un deuxième dispositif (62) de traitement d'image qui forme un signal (RGB) de couleur d'image à partir du premier signal (R) d'intensité d'image et du deuxième signal (G, B) d'intensité d'image, le signal (RGB) de couleur d'image représentant des propriétés optiques de l'objet (19).

2. Dispositif de caméra (30) selon la revendication précédente, dans lequel la première plage (83, 84) de longueurs d'onde est située au moins en partie à l'intérieur de la plage (80) des longueurs d'onde visibles pour l'oeil humain.

3. Dispositif de caméra (30) selon l'une des revendications précédentes, dans lequel le deuxième dispositif (62) de traitement de signal est configuré pour former le signal (RGB) de couleur d'image sans recourir au signal (D) de distance d'image.

4. Dispositif de caméra (30) selon l'une des revendications précédentes, dans lequel le premier capteur (53) d'image et le deuxième capteur (51, 52) d'image présentent différentes résolutions, et présentant en outre un dispositif (64) de traitement de signal qui adapte l'une à l'autre une première résolution du premier signal (R) d'intensité d'image et une deuxième résolution du deuxième signal (G, B) d'intensité d'image.

5. Dispositif de caméra (30) selon la revendication précédente, dans lequel l'autre dispositif (64) de traitement d'image est configuré pour interpoler le signal (R) d'intensité d'image qui présente la résolution la plus basse pour des positions d'éléments d'image du signal (G, B) d'intensité d'image présentant la résolution plus élevée, en vue de réaliser l'adaptation à différentes résolutions.

6. Dispositif de caméra (30) selon la revendication précédente, dans lequel l'autre dispositif (64) de traitement d'image est configuré pour utiliser pour l'interpolation des informations provenant du signal (G, B) d'intensité d'image qui présente la résolution plus élevée.

7. Endoscope (10) ou exoscope doté d'un dispositif de caméra (30) selon l'une des revendications précédentes.

8. Procédé de fabrication d'un dispositif de caméra (30) destiné à saisir des propriétés optiques dans plusieurs plages différentes (81, 82, 83) de longueurs d'onde et des propriétés structurelles spatiales d'un objet (19), le procédé présentant les étapes suivantes :
prévoir (101) un diviseur (40) de faisceau qui divise la lumière en plusieurs plages différentes (81, 82, 83) de longueurs d'onde, un capteur (51, 52, 59) d'image photosensible étant prévu sur le diviseur de faisceau (40) pour chacune des différentes plages (81, 82, 83) de longueurs d'onde,
retirer (102) un capteur (59) d'image du diviseur (40) de faisceau,
remplacer (103) le capteur (59) d'image qui a été retiré par un premier capteur (53) d'image qui, du fait de sa sensibilité modulable dans le temps, est configuré pour saisir la distance d'un objet (19) par rapport au dispositif de caméra (30) à partir du temps de parcours de la lumière (L, M, N) et qui est sensible à la phase,
un deuxième capteur (51, 52) d'image restant sur le diviseur (40) de faisceau,
le premier capteur (53) d'image étant sensible dans la plage (83) de longueurs d'onde qui était associée au capteur (59) d'image qui a été retiré, et présentant en outre les étapes suivantes :
prévoir (104) un premier dispositif (61) de traitement d'image qui reçoit un premier signal (T) du premier capteur (53)- d'image et qui forme un premier signal (R) d'intensité d'image et un signal (D) de distance d'image en recourant au premier signal (T) du premier capteur (53) d'image,
prévoir un deuxième dispositif (62) de traitement de signal qui reçoit le premier signal (R) d'intensité d'image, qui reçoit du deuxième capteur (61, 62) d'image un deuxième signal (B, G) d'intensité d'image dans une deuxième plage de longueurs d'onde prédéterminée parmi les différentes plages de longueurs d'onde et qui forme un signal (RGB) de couleur d'image en recourant au premier signal (R) d'intensité d'image et au deuxième signal (G, B) d'intensité d'image.

9. Procédé selon la revendication 8, présentant en outre l'étape suivants :
prévoir (107) un autre dispositif (64) de traitement de signal qui adapte l'une à l'autre une première résolution du premier signal (R) d'intensité d'image et une deuxième résolution du deuxième signal (G, B) d'intensité d'image.

10. Procédé de saisie de propriétés optiques dans plusieurs plages (81, 82, 83) différentes de longueurs d'onde et de propriétés structurelles spatiales d'un objet (19), le procédé présentant les étapes suivantes :
éclairer (114) l'objet (19) avec une lumière d'éclairage (L, M) modulée dans le temps de manière prédéterminée,
saisir (115) un premier signal (T) de capteur associé à une première plage (83, 84) de longueurs d'onde prédéterminée parmi les différentes plages de longueurs d'onde, au moyen d'un premier capteur (53) d'image dont la sensibilité est modulée dans le temps et sensible à la phase,
former (116) un premier signal (R) d'intensité d'image en recourant au premier signal (T) de capteur,
former (117) un signal (D) de distance d'image en recourant à une information concernant la modulation dans le temps de la lumière d'éclairage (L, M) et du premier signal (T) de capteur,
saisir (113) un deuxième signal (G, B) d'intensité d'image qui se rapporte à une deuxième plage (81, 82) de longueurs d'onde prédéterminée parmi les différentes plages de longueurs d'onde, au moyen d'un deuxième capteur (51, 52) d'image,
former (120) un signal (RGB) de couleur d'image en recourant au premier signal (R) d'intensité d'image et au deuxième signal (G, B) d'intensité d'image,
le signal (RGB) de couleur d'image représentant des propriétés optiques de l'objet (19) et le signal (R) de distance d'image des propriétés structurelles spatiales de l'objet (19).

11. Procédé selon la revendication précédente, dans lequel le signal (RGB) de couleur d'image est formé (118) sans recourir au signal (D) de distance d'image.

12. Procédé selon l'une des revendications 10 à 11, dans lequel les résolutions du premier signal (R) d'intensité d'image et du deuxième signal (G, B) d'intensité d'image sont différentes l'une de l'autre, et présentant en outre l'étape qui consiste à adapter (118) les résolutions l'une à l'autre.

13. Procédé selon la revendication précédente, dans lequel l'adaptation (118) des résolutions comporte une interpolation (119) du signal (R) d'intensité d'image présentant la résolution plus basse pour des positions d'éléments du signal d'image (G, B) de résolution plus élevée, en utilisant des informations provenant du signal (G, B) d'image présentant la résolution plus élevée.
